# EUROPÄISCHE PATENTSCHRIFT

(11) **EP 0 461 502 B1**
(45) Veröffentlichungstag und Bekanntmachung des Hinweises auf die Patenterteilung: **28.08.1996**
(21) Anmeldenummer: 91108991.0
(22) Anmeldetag: 01.06.1991
(51) Int. Cl.: C07D 521/00, C07D 303/08, C07D 303/22, C07D 407/04, A01N 43/653

(54) **Azolyl-propanol-Derivate**
Azolyl propanol derivatives
Dérivés d'azolyl-propanole

(30) Priorität: 13.06.1990 DE 4018927
(43) Veröffentlichungstag der Anmeldung: 18.12.1991
(73) Patentinhaber: BAYER AG, 51368 Leverkusen (DE)
(72) Erfinder: Scherkenbeck, Jürgen, Dr., W-5090 Leverkusen 3 (DE); Himmler, Thomas, Dr., W-5000 Köln 80 (DE); Stroech, Klaus, Dr., W-5650 Solingen 19 (DE); Dutzmann, Stefan, Dr., W-4010 Hilden (DE); Hänssler, Gerd, Dr., W-5090 Leverkusen 3 (DE); Dehne, Heinz-Wilhelm, Dr., W-4019 Monheim (DE)

(56) Entgegenhaltungen:
- EP-A- 0 297 345
- EP-A- 0 351 649

## Beschreibung

Die vorliegende Erfindung betrifft neue Azolyl-propanol-Derivate, ein Verfahren zu deren Herstellung und deren Verwendung als Fungizide.

Es ist bereits bekannt geworden, daß bestimmte Azolyl-propanol-Derivate fungizide Eigenschaften besitzen (vgl. EP-OS 0 015 756, EP-A 0 351 649 und EP-OS 0 297 345). Die Wirksamkeit dieser Stoffe ist gut; sie läßt allerdings bei niedrigen Aufwandmengen in manchen Fällen zu wünschen übrig.

Es wurden nun neue Azolyl-propanol-Derivate der Formel 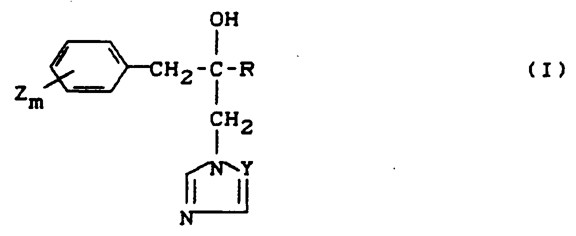 in welcher
- R: für Halogenalkyl mit 1 bis 18 Kohlenstoffatomen und 1 bis 12 Halogenatomen, Halogenalkenyl mit 2 bis 18 Kohlenstoffatomen und 1 bis 12 Halogenatomen oder einen Rest der Formel 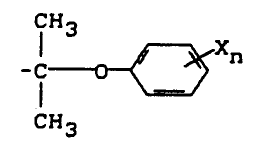 steht, worin
- X: für Alkyl mit 1 bis 6 Kohlenstoffatomen, Halogen oder Halogenalkyl mit 1 bis 4 Kohlenstoffatomen und 1 bis 5 Halogenatomen steht und
- n: für die Zahlen 0, 1, 2 oder 3 steht, oder
- R: für einen Rest der Formel 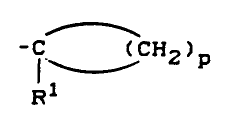 steht, worin
- R¹: für n-Propyl, Vinyl oder für gegebenenfalls durch Halogen und/oder Alkyl mit 1 bis 4 Kohlenstoffatomen substituiertes Phenyl steht und
- p: für die Zahlen 3, 4 oder 5 steht, oder
- R: für einen Rest der Formel 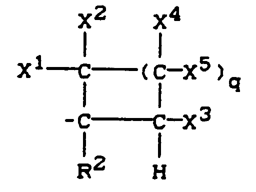 steht, worin
- R²: für Wasserstoff, Halogen, Methyl oder Ethyl steht,
- X¹: für Halogen oder Trifluormethyl steht,
- X²: für Halogen oder Trifluormethyl steht,
- X³: für Wasserstoff oder Halogen steht,
- X⁴: für Wasserstoff oder Halogen steht,
- X⁵: für Wasserstoff oder Halogen steht und
- q: für die Zahlen 0, 1 oder 2 steht,
oder
- R: für einen Rest der Formel 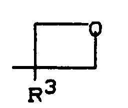 steht, worin
- R³: für Alkyl mit 1 bis 4 Kohlenstoffatomen oder für Halogen steht,
- Y: für Stickstoff oder eine CH-Gruppe steht,
- Z: für Halogen, Alkyl mit 1 bis 6 Kohlenstoffatomen, Halogenalkyl mit 1 bis 6 Kohlenstoffatomen und 1 bis 13 Halogenatomen, Alkoxy mit 1 bis 6 Kohlenstoffatomen, Halogenalkoxy mit 1 bis 6 Kohlenstoffatomen und 1 bis 13 Halogenatomen, Cyano, gegebenenfalls durch Halogen substituiertes Phenoxy oder für Aryl mit 6 bis 10 Kohlenstoffatomen steht, wobei jeder der Aryl-Reste durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen und/oder Halogenalkoxy mit 1 bis 4 Kohlenstoffatomen und 1 bis 5 Halogenatomen substituiert sein kann, und
- m: für die Zahlen 0, 1, 2 oder 3 steht,
sowie deren Säureadditions-Salze und Metallsalz-Komplexe gefunden.

Weiterhin wurde gefunden, daß man Azolyl-propanol-Derivate der Formel (I) sowie deren Säureadditions-Salze und Metallsalz-Komplexe erhält, wenn man Oxirane der Formel 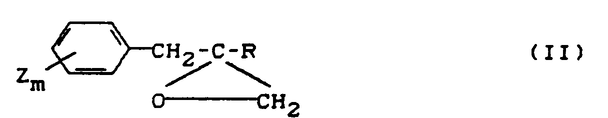 in welcher
R, Z und m die oben angegebenen Bedeutungen haben,
mit Azolen der Formel 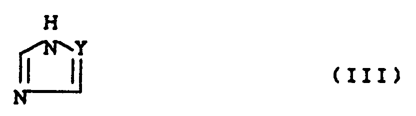 in welcher
- Y: die oben angegebene Bedeutung hat,
gegebenenfalls in Gegenwart eines Säurebindemittels und in Gegenwart eines Verdünnungsmittels umsetzt
und gegebenenfalls anschließend an die so erhaltenen Verbindungen der Formel (I) eine Säure oder ein Metallsalz addiert.

Schließlich wurde gefunden, daß die neuen Azolyl-propanol-Derivate der Formel (I) sowie deren Säureadditions-Salze und Metallsalz-Komplexe sehr gute fungizide Eigenschaften besitzen.

Die erfindungsgemäßen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Überraschenderweise besitzen die erfindungsgemäßen Stoffe bessere fungizide Eigenschaften als die konstitutionell ähnlichsten vorbekannten Verbindungen gleicher Wirkungsrichtung.

Die erfindungsgemäßen Azolyl-propanol-Derivate sind durch die Formel (I) allgemein definiert.

In den Verbindungen der oben angegebenen Formel steht
- R: vorzugsweise für Halogenalkyl mit 1 bis 6 Kohlenstoffatomen und 1 bis 6 Fluor-, Chlor- und/oder Bromatomen oder Halogenalkenyl mit 2 bis 8 Kohlenstoffatomen und 1 bis 6 Fluor-, Chlor- und/oder Bromatomen,
besonders bevorzugt für Halogenalkyl mit 1 bis 4 Kohlenstoffatomen und 1 bis 5 Fluor-, Chlor-und/oder Bromatomen oder für Halogenalkenyl mit 2 bis 6 Kohlenstoffatomen und 1 bis 5 Fluor-, Chlor- und/oder Bromatomen.

Ausßerdem steht
- R: für einen Rest der Formel 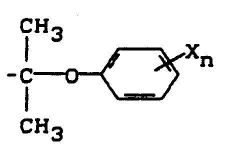 worin
- X: vorzugsweise für Alkyl mit 1 bis 4 Kohlenstoffatomen, Fluor, Chlor oder Halogenalkyl mit 1 oder 2 Kohlenstoffatomen und 1 bis 3 Fluor- und/oder Chloratomen steht und besonders bevorzugt für Methyl, Ethyl, Fluor, Chlor, Trifluormethyl oder Trichlormethyl steht und
- n: für die Zahlen 0, 1 oder 2 steht.

Weiterhin steht
- R: für einen Rest der Formel 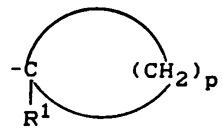 worin
- R¹: vorzugsweise für n-Propyl, Vinyl oder für gegebenenfalls einfach oder zweifach, gleichartig oder verschieden durch Fluor, Chlor, Methyl und/oder Ethyl substituiertes Phenyl steht und
- p: für die Zahlen 3, 4 oder 5 steht.

Ferner steht
- R: für einen Rest der Formel 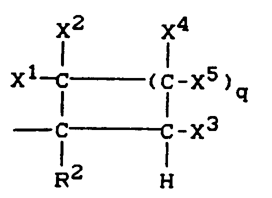 worin
- R²: vorzugsweise für Wasserstoff, Fluor, Chlor, Brom, Methyl oder Ethyl steht,
- X¹: vorzugsweise für Fluor, Chlor oder Trifluormethyl steht,
- X²: vorzugsweise für Fluor, Chlor oder Trifluormethyl steht,
- X³: vorzugsweise für Wasserstoff, Fluor oder Chlor steht,
- X⁴: vorzugsweise für Wasserstoff, Fluor oder Chlor steht,
- X⁵: vorzugsweise für Wasserstoff, Fluor oder Chlor steht, und
- q: für die Zahlen 0, 1 oder 2 steht.

Schließlich steht
- R: für einen Rest der Formel 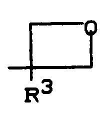 worin
- R³: vorzugsweise für Methyl, Ethyl, Fluor, Chlor oder Brom steht.
- Y: steht für Stickstoff oder eine CH-Gruppe.
- Z: steht vorzugsweise für Fluor, Chlor, Brom, Alkyl mit 1 bis 4 Kohlenstoffatomen, Halogenalkyl mit 1 bis 4 Kohlenstoffatomen und 1 bis 5 Fluor- und/oder Chloratomen, Alkoxy mit 1 bis 4 Kohlenstoffatomen, Halogenalkoxy mit 1 bis 4 Kohlenstoffatomen und 1 bis 5 Fluor- und/oder Chloratomen, Cyano, gegebenenfalls einfach bis dreifach durch Fluor und/oder Chlor substituiertes Phenoxy, für Phenyl oder Naphthyl, wobei jeder der beiden zuletzt genannten Reste einfach bis dreifach, gleichartig oder verschieden substituiert sein kann durch Fluor, Chlor, Alkyl mit 1 bis 3 Kohlenstoffatomen, Halogenalkyl mit 1 oder 2 Kohlenstoffatomen und 1 bis 5 Fluor und/oder Chloratomen, Alkoxy mit 1 bis 3 Kohlenstoffatomen oder durch Halogenalkoxy mit 1 oder 2 Kohlenstoffatomen und 1 bis 5 Fluor- und/oder Chloratomen.
- Z: steht besonders bevorzugt für Fluor, Chlor, Brom, Methyl, Ethyl, Trifluormethyl, Trichlormethyl, Methoxy, Ethoxy, Trifluormethoxy, Trichlormethoxy, Cyano, gegebenenfalls durch Fluor und/oder Chlor einfach oder zweifach substituiertes Phenoxy, gegebenenfalls einfach oder zweifach, gleichartig oder verschieden durch Fluor, Chlor, Methyl, Ethyl, Trifluormethyl, Trichlormethyl, Methoxy, Ethoxy, Trifluormethoxy oder Trichlormethoxy substituiertes Phenyl.
- m: steht für die Zahlen 0, 1, 2 oder 3.

Wenn m in der Formel (I) für die Zahlen 2 oder 3 steht, können die für Z stehenden Reste gleich oder verschieden sein.

Als Beispiele für Azolyl-propanol-Derivate der Formel (I) seien die in der folgenden Tabelle aufgeführten Stoffe genannt.

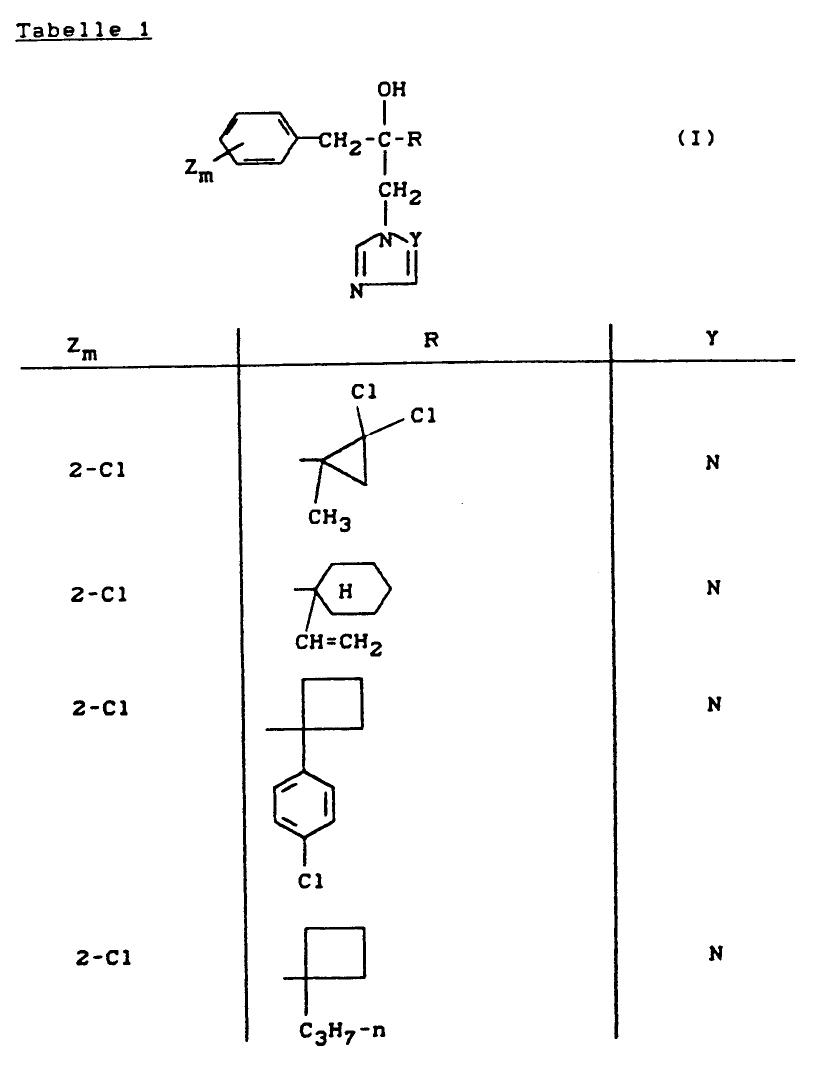

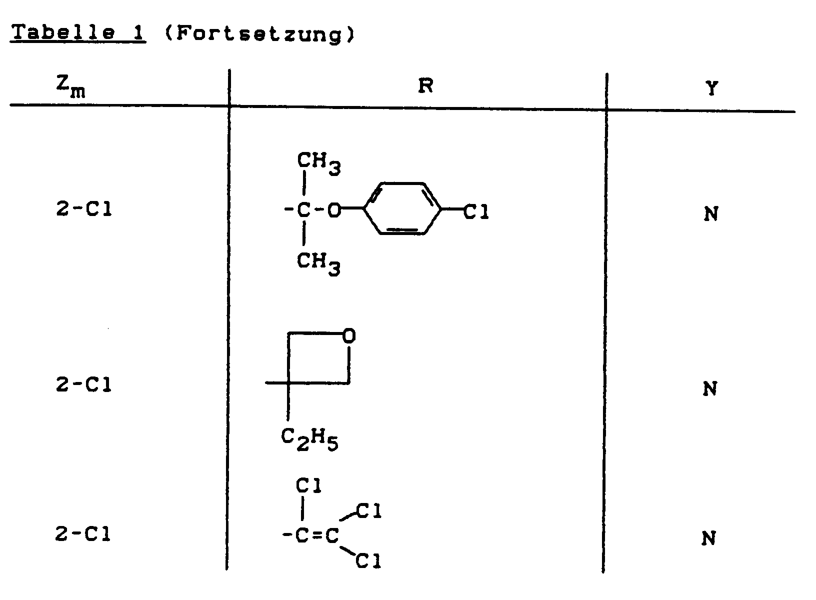

Bevorzugte erfindungsgemäße Stoffe sind auch Additionsprodukte aus Säuren und denjenigen Azolyl-propanol-Derivaten der Formel (I), in denen R, Y, Z und m die Bedeutungen haben, die bereits vorzugsweise für diese Reste und den Index m genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Azolyl-propanol-Derivaten der Formel (I), in denen R, Y, Z und m die Bedeutungen haben, die bereits vorzugsweise für diese Reste und den Index m genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man 2-(4-Fluor-benzyl)-2-(2,2-difluor-1-methyl-cyclopropyl)-oxiran und 1,2,4-Triazol als Ausgangsstoffe, so kann der Verlauf des erfindungsgemäßen Verfahrens durch das folgende Formelschema veranschaulicht werden.

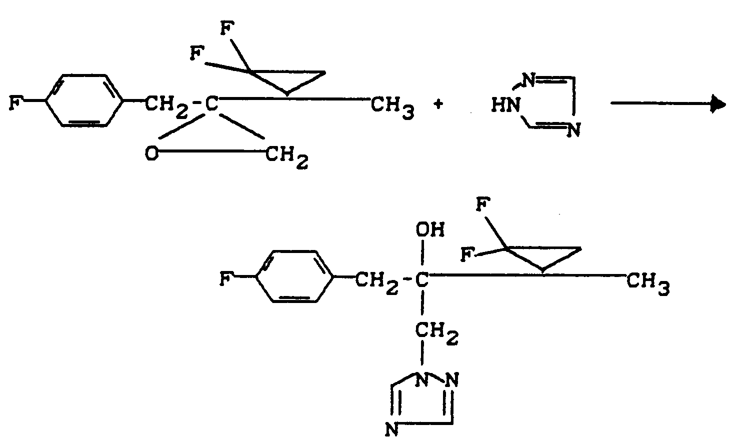

Die bei dem erfindungsgemäßen Verfahren als Ausgangsstoffe benötigten Oxirane sind durch die Formel (II) allgemein definiert.

Die Oxirane der Formel (II) sind bisher noch nicht bekannt. Sie lassen sich herstellen, indem man Benzylketone der Formel 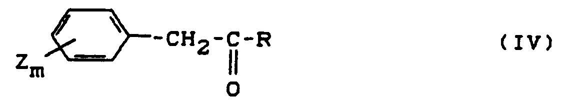 in welcher
R, Z und m die oben angegebenen Bedeutungen haben,
entweder
α) mit Dimethyloxosulfonium-methylid derFormel 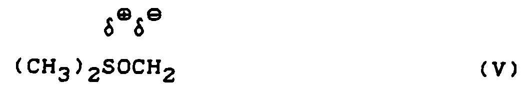 oder
β) mit Dimethylsulfonium-methylid der Formel 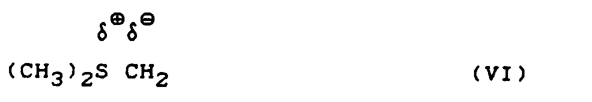 in Gegenwart eines Verdünnungsmittels umsetzt.

Die Benzylketone der Formel (IV) sind bisher nur teilweise bekannt. Sie lassen sich herstellen, indem man
a) in einer ersten Stufe Benzylchloride der Formel 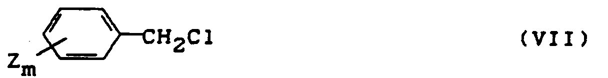 in welcher
   Z und m die oben angegebenen Bedeutungen haben,
   mit einem Überschuß an Zinkpulver in Gegenwart eines Verdünnungsmittels bei Temperaturen zwischen 30°C und 200°C unter Schutzgasatmosphäre umsetzt, das überschüssige Zinkpulver abtrennt und dann
b) in einer zweiten Stufe die entstandenen Benzylderivate der Formel 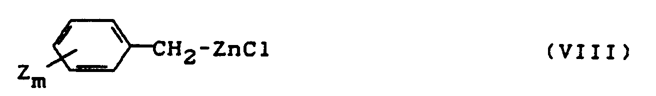 in welcher
   Z und m die oben angegebenen Bedeutungen haben,
   mit Säurechloriden der Formel

   R-CO-Cl (IX)

   in welcher
   R die oben angegebene Bedeutung hat,
in Gegenwart eines Palladiumkatalysators und in Gegenwart eines Verdünnungsmittels bei Temperaturen zwischen 0°C und 150°C unter Schutzgasatmosphäre umsetzt.

Die bei der Durchführung des obigen Verfahrens zur Herstellung von Benzylketonen der Formel (IV) als Ausgangsstoffe benötigten Benzylchloride der Formel (VII) sind bekannt oder lassen sich nach prinzipiell bekannten Verfahren in einfacher Weise herstellen.

Auch die bei der Durchführung des obigen Verfahrens zur Herstellung von Benzylketonen der Formel (IV) als Reaktionskomponenten benötigten Säurechloride der Formel (IX) sind bekannt oder lassen sich nach prinzipiell bekannten Verfahren in einfacher Weise herstellen.

Das Zinkpulver kann bei der Durchführung des obigen Verfahrens zur Herstellung von Benzylketonen der Formel (IV) in verschiedenen Korngrößen eingesetzt werden. Vorzugsweise verwendbar ist Zinkstaub oder Zinkpulver kleiner Teilchengröße.

Als Palladiumkatalysatoren kommen bei der Durchführung des obigen Verfahrens zur Herstellung von Benzylketonen der Formel (IV) kommen alle für derartige Zwecke üblichen Palladium(II)-salze, Palladium(II)-komplexe und Palladium(O)-komplexe in Betracht. Vorzugsweise verwendbar sind Palladium(II)-chlorid, Palladium(II)-acetat, Bis(tri-phenylphosphin)-palladium(II)-chlorid, Bis(benzonitril)-palladium(II)-chlorid und Tetrakis(tri-phenylphosphin)-palladium(O).

Als Verdünnungsmittel können bei der Herstellung von Benzylketonen der Formel (IV) nach dem obigen Verfahren sowohl bei der Durchführung der ersten als auch der zweiten Stufe alle üblichen inerten, organischen Solventien eingesetzt werden. Vorzugsweise verwendbar sind Ether, wie Diethylether, tert.-Butylmethyl-ether, tert.-Amyl-methyl-ether, Tetrahydrofuran, 2,5-Dimethyl-tetrahydrofuran, Ethylenglykol-dimethylether, Ethylenglykoltert.-butyl-methyl-ether, Diethylenglykol-diethylether und Dioxan, ferner Nitrile, wie Acetonitril, und außerdem Amide, wie Dimethylformamid und Dimethylacetamid. Die Lösungsmittel werden vorzugsweise in trockener Form eingesetzt.

Als Schutzgase kommen bei der Durchführung des Verfahrens zur Herstellung von Benzylketonen der Formel (IV) sowohl in der ersten als auch in der zweiten Stufe alle üblichen inerten Gase in Frage. Vorzugsweise verwendbar sind Helium, Argon und Stickstoff.

Die Reaktionstemperaturen können bei der Durchführung des Verfahrens zur Herstellung von Benzylketonen der Formel (IV) sowohl in der ersten als auch in der zweiten Stufe innerhalb eines größeren Bereiches variiert werden. In der ersten Stufe arbeitet man im allgemeinen bei Temperaturen zwischen 30°C und 200°C, vorzugsweise zwischen 50°C und 150°C, in der zweiten Stufe arbeitet man im allgemeinen bei Temperaturen zwischen 0°C und 150°C, vorzugsweise zwischen 20°C und 100°C.

Auch die Reaktionszeiten können im Falle des obigen Verfahrens zur Herstellung von Benzylketonen der Formel (IV) sowohl bei der Durchführung der ersten als auch der zweiten Stufe innerhalb eines größeren Bereiches variiert werden. In der ersten Stufe liegen die Reaktionszeiten im allgemeinen zwichen 0,5 und 4 Stunden, vorzugsweise zwischen 1 und 3 Stunden. In der zweiten Stufe liegen die Reaktionszeiten im allgemeinen zwischen 0,1 und 5 Stunden, vorzugsweise zwischen 0,5 und 3 Stunden.

Das Verfahren zur Herstellung von Benzylketonen der Formel (IV) wird im allgemeinen unter Normaldruck durchgeführt. Es ist aber auch möglich, unter vermindertem oder erhöhtem Druck zu arbeiten.

Bei der Durchführung der ersten Stufe des obigen Verfahrens zur Herstellung von Benzylketonen der Formel (IV) setzt man das Benzylchlorid der Formel (VII) mit einem Überschuß an Zink um, Auf 1 Mol an Benzylchlorid der Formel (VII) setzt man im allgemeinen zwischen 1,05 und 3 Mol, vorzugsweise zwischen 1,1 und 2,5 Mol, besonders bevorzugt zwischen 1,25 und 2,0 Mol an Zink ein. Bei der Durchführung der zweiten Stufe des obigen Verfahrens zur Herstellung von Benzylketonen der Formel (IV) kann die Menge an Palladiumkatalysator innerhalb eines bestimmten Bereiches variiert werden. Auf 1 Mol an Säurechlorid der Formel (IX) setzt man im allgemeinen zwischen 0,001 und 1 Mol-%, vorzugsweise Zwischen 0,0025 und 0,1 Mol-%, besonders bevorzugt zwischen 0,005 und 0,05 Mol-% an Palladiumkatalysator ein.

Das Verhältnis von Benzylchlorid der Formel (VII) zu Säurechlorid der Formel (IX) kann bei der Durchführung des obigen Verfahrens zur Herstellung von Benzylketonen der Formel (IV) innerhalb eines größeren Bereiches variiert werden. Auf 1 Mol an Säurechlorid der Formel (IX) setzt man im allgemeinen zwischen 1,01 und 1,25 Mol, vorzugsweise zwischen 1,05 und 1,2 Mol an Benzylchlorid der Formel (VII) ein.

Nach Beendigung der ersten Stufe des obigen Verfahrens zur Herstellung von Benzylketonen der Formel (IV) kann das überschüssige Zink nach üblichen Methoden abgetrennt werden. Die Entfernung des Zinks kann z.B. durch Filtration oder Zentrifugieren erfolgen, oder auch dadurch geschehen, daß man nach dem Absetzen des Zinks die überstehende Lösung abpumpt.

Bei der Durchführung des obigen Verfahrens zur Herstellung von Benzylketonen der Formel (IV) geht man im einzelnen so vor, daß man zunächst das Benzylchlorid der Formel (VII) mit Zink in einem geeigneten Verdünnungsmittel unter Schutzgasatmosphäre umsetzt, dann das überschüssige Zink abtrennt, zu der verbleibenden Lösung das Säurechlorid der Formel (IX) und den Palladiumkatalysator hinzufügt und bis zur Beendigung der Umsetzung reagieren läßt. Die Aufarbeitung erfolgt nach üblichen Methoden. Im allgemeinen geht man so vor, daß man das Reaktionsgemisch nach gegebenenfalls vorheriger Filtration sowie gegebenenfalls nach dem Verdünnen mit einem mit Wasser wenig mischbaren organischen Lösungsmittel mit Wasser und gegebenenfalls mit verdünnter, wäßriger Mineralsäure versetzt, die organische Phase abtrennt, trocknet und einengt. Das verbleibende Produkt kann nach üblichen Methoden, wie z.B. Destillation oder Umkristallisation, von eventuell noch vorhandenen Verunreinigungen befreit werden.

Das bei der Durchführung der Variante (α) des Verfahrens zur Herstellung von Oxiranen der Formel (II) als Reaktionskomponente benötigte Dimethyloxosulfonium-methylid der Formel (V) ist bekannt (vgl. J Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, oder durch Umsetzung von Trimethyloxosulfoniumchlorid mit wäßriger Natronlauge, jeweils in Gegenwart eines Verdünnungsmittels erzeugt.

Das bei der Durchführung der Variante (β) des Verfahrens zur Herstellung von Oxiranen der Formel (II) außerdem als Reaktionskomponente in Betracht kommende Dimethylsulfonium-methylid der Formel (VI) ist ebenfalls bekannt (vgl. Heterocycles 8, 397 (1977)). Es wird bei der obigen Umsetzung ebenfalls in frisch hergestelltem Zustand eingesetzt, indem man es in situ z.B. aus Trimethylsulfonium-halogenid oder Trimethylsulfonium-methylsulfat, in Gegenwart einer starken Base, wie z.B. Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des obigen Verfahrens zur Herstellung von Oxiranen der Formel (II) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung des obigen Verfahrens zur Herstellung von Oxiranen der Formel (II) in einem größeren Bereich variiert werden. Im allgemeinen arbeitet man bei Temperaturen zwischen 0°C und 100°C, vorzugsweise zwischen 10°C und 60°C.

Bei der Durchführung des obigen Verfahrens zur Herstellung von Oxiranen der Formel (II) setzt man auf 1 Mol an Benzylketon der Formel (IV) im allgemeinen 1 bis 3 Mol an Dimethyloxosulfonium-methylid der Formel (V) bzw. an Dimethylsulfonium-methylid der Formel (VI) ein.

Die Isolierung der Oxirane der Formel (II) erfolgt nach üblichen Methoden.

Als Säurebindemittel kommen bei der Durchführung des erfindungsgemäßen Verfahrens alle üblichen anorganischen und organischen Basen in Frage. Vorzugsweise verwendbar sind Alkalicarbonate, wie Natrium- und Kaliumcarbonat, ferner Alkalimetallhydroxide, wie Natrium- und Kaliumhydroxid, außerdem Alkalimetallalkoholate, wie Natrium- und Kaliummethylat und -ethylat sowie Kalium-tert.-butylat, und weiterhin niedere tertiäre Alkylamine, Cycloalkylamine und Aralkylamine, wie insbesondere Triethylamin.

Als Verdünnungsmittel kommen bei der Durchführung des erfindungsgemäßen Verfahrens alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare Lösungsmittel, wie Dimethylsulfoxid und Hexamethylphosphorsäuretriamid.

Die Reaktionstemperaturen können bei der Durchführung des erfindungsgemäßen Verfahrens in einem größeren Bereich variiert werden. Im allgemeinen arbeitet man bei Temperaturen zwischen 0°C und 200°C, vorzugsweise zwischen 50°C und 150°C.

Bei der Durchführung des erfindungsgemäßen Verfahrens setzt man vorzugsweise auf 1 Mol an Oxiran der Formel (II) 1 bis 4 Mol an Azol der Formel (III) und 1 bis 2 Mol an Base ein. Die Isolierung der Endprodukte erfolgt in üblicher Weise.

Die erfindungsgemäßen Azolyl-propanol-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Leptosphaeria nodorum, Erysiphe und Pseudocercosporella. Außerdem zeigen die erfindungsgemäßen Stoffe eine sehr gute Wirkung gegen Venturia, Sphaerotheca und Botrytis.

Die erfindungsgemäßen Stoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage; z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Behandlung von Saatgut werden im allgemeinen Wirkstoffmengen von 0,001 bis 50 g je Kilogramm Saatgut, vorzugsweise 0,01 bis 10 g benötigt. Bei der Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Stoffe geht aus den folgenden Beispielen hervor.

### Herstellungsbeispiele

### Beispiel 1

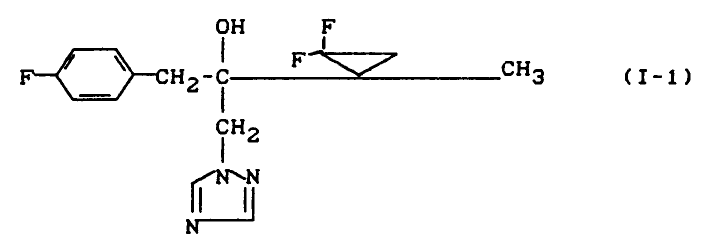

Eine Lösung von 6,3 g (0,026 Mol) 2-(4-Fluorbenzyl)-2-(2,2-difluor-1-methyl-cyclopropyl)-oxiran in 5 ml Dimethylformamid wird bei 80°C unter Rühren in ein Gemisch aus 4,6 g (0,066 Mol) 1,2,4-Triazol und 0,5 g (0,0045 Mol) Kalium-tert.-butylat in 10 ml Dimethylformamid eingetropft. Nach beendeter Zugabe wird das Reaktionsgemisch 8 Stunden bei 80°C gerührt. Anschließend wird das Lösungsmittel unter vermindertem Druck abgezogen und der verbleibende Rückstand in Essigsäureethylester gelöst. Die organische Lösung wird mit Wasser gewaschen, über Natriumsulfat getrocknet und unter vermindertem Druck eingeengt. Der Rückstand wird mit Dichlormethan als Laufmittel an Kieselgel chromatographiert. Nach dem Eindampfen des Eluates erhält man 1,7 g (21 % der Theorie) an 1-(4-Fluorphenyl)-2-(2,2-difluor-1-methyl-cyclopropyl)-3-(1,2,4-triazol-1-yl)-propan-2-ol in Form einer Festsubstanz mit dem Schmelzpunkt 114°C.

### Herstellung von Ausgangssubstanzen:

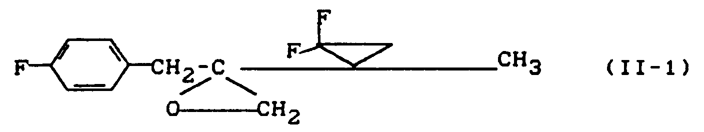

In ein Gemisch aus 5,31 g (0,0241 Mol) Trimethylsulfoxoniumiodid und 0,79 g (0,0329 Mol) Natriumhydrid (80 %ig) werden bei 10°C unter Stickstoffatmosphäre 20 ml absolutes Dimethylsulfoxid eingetropft. Nach beendeter Zugabe läßt man innerhalb von 10 Minuten auf Raumtemperatur erwärmen und tropft dann unter Rühren eine Lösung von 5 g (0,0219 Mol) 4-Fluorbenzyl-(2,2-difluor-1-methyl-cyclopropyl)-keton in 10 ml absolutem Dimethylsulfoxid hinzu. Es wird 4 Stunden bei 40°C nachgerührt. Danach wird das Reaktionsgemisch auf Eiswasser gegossen. Das entstehende Gemisch wird dreimal mit Cyclohexan extrahiert. Die vereinigten organischen Phasen werden über Natriumsulfat getrocknet und unter vermindertem Druck eingeengt. Man erhält auf diese Weise 6,3 g an 2-(4-Fluor-benzyl)-2-(2,2-difluor-1-methylcyclopropyl)-oxiran, das ohne zusätzliche Reinigung für die weitere Umsetzung verwendet wird.

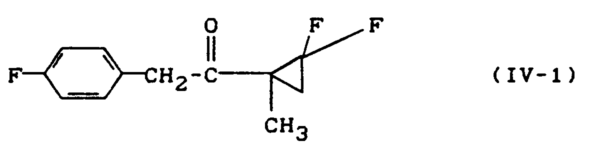

Ein Gemisch aus 6,5 g (100 mmol) Zinkpulver, 9,55 g (66 mmol) 4-Fluorbenzylchlorid und 75 ml trockenem Ethylenglykol-dimethylether wird unter Stickstoffatmosphäre 1 Stunde unter Rückfluß erhitzt. Anschließend wird das Reaktionsgemisch unter Stickstoff filtriert. Das Filtrat wird mit 6,9 g (44,6 mmol) 2,2-Difluor-1-methyl-cyclopropan-carbonsäurechlorid und 21 mg (0,07 Mol-%) Bis-(triphenylphosphin)palladium(II)-chlorid versetzt und unter Stickstoffatmosphäre 1,5 Stunden unter Rückfluß erhitzt. Anschließend wird das Reaktionsgemisch unter Stickstoff filtriert. Man verdünnt das Filtrat mit Toluol, schüttelt nacheinander mit verdünnter, wäßriger Salzsäure und Wasser aus, trocknet die organische Phase und engt durch Abziehen des Lösungsmittels unter vermindertem Druck ein. Der verbleibende Rückstand wird einer Destillation unterworfen. Man erhält auf diese Weise 8,8 g (85 % der Theorie) an 4-Fluorbenzyl-(2,2-difluor-1-methyl)-cyclopropyl-keton in Form eines Öles.

¹H-NMR(200 MHz, CDCl₃): δ = 1,2-1,35 (m; 1H), 1,5-1,6 (m; 3H), 2,25-2,4 (m; 1H), 3,8 (d, J ≃ 17 Hz; 1H), 3,84 (d, J ≃ 17 Hz; 1H), 6,9-7,2 (m; 4H) ppm.

Nach der im Beispiel 1 angegebenen Methode werden auch die in den folgenden Beispielen aufgeführten Stoffe hergestellt.

### Beispiel 2

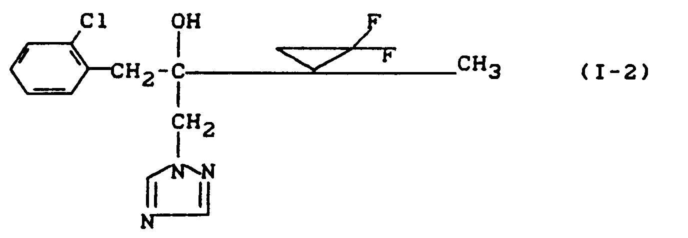

¹H-NMR (250 MHz, CDCl₃):
- δ =: 1,5 (t; 3H), 3,3 (AB-System; 2H), 4,3 (AB-System; 2H); 7,2-7,6 (H arom.), 7,9 (s, 1H), 8,1 (s; 1H)

### Beispiel 3

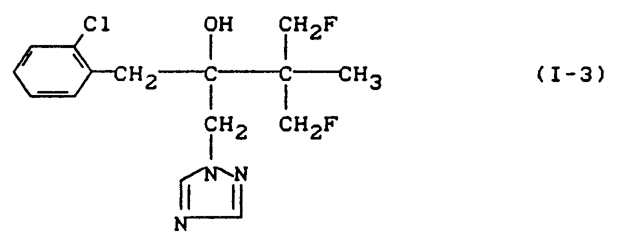

¹H-NMR (250 MHz, CDCl₃):
- δ =: 1,0 (t; 3H), 3,3 (A,B-System; 2H), 4,2-4,8 (m), 7,2-7,5 (H arom.), 7,8 (s; 1H), 7,9 (s, 1H)

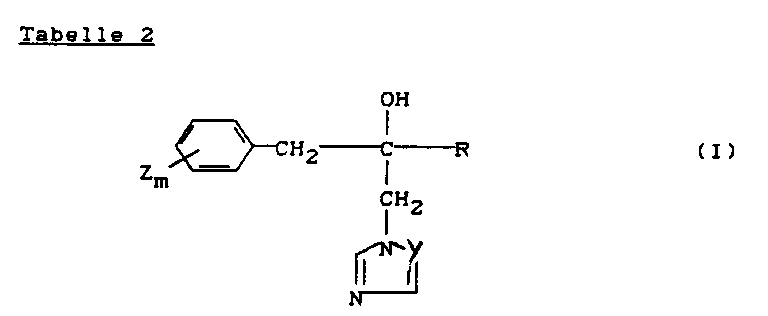

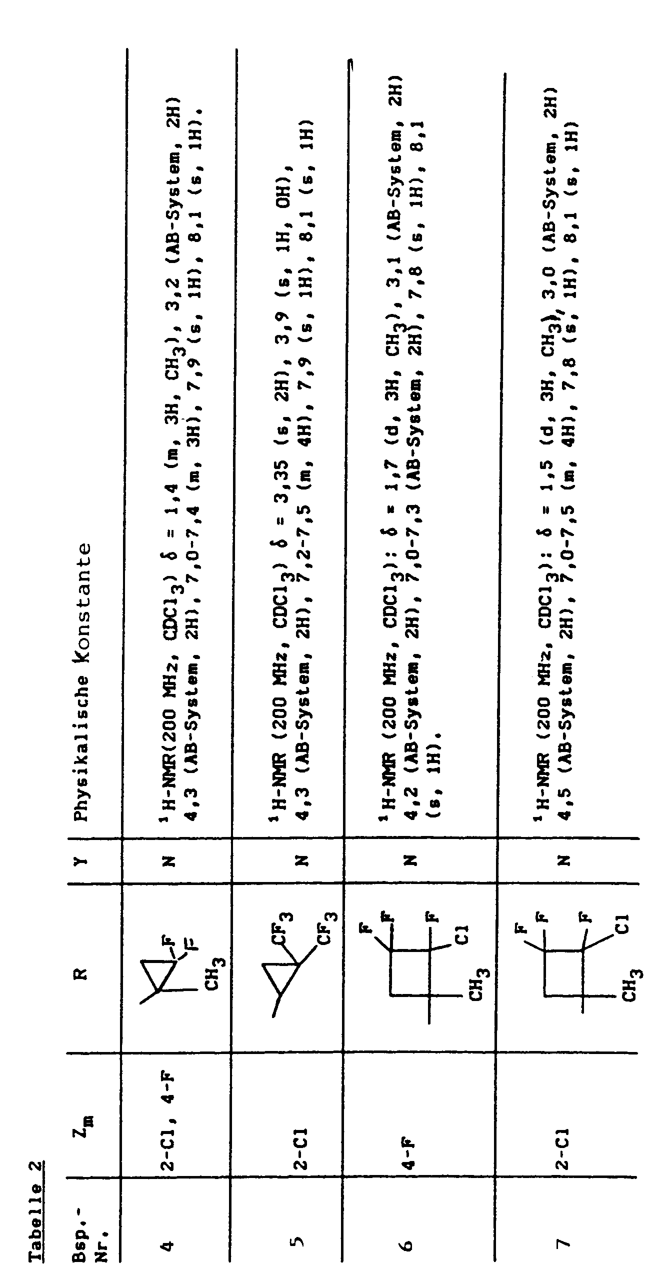

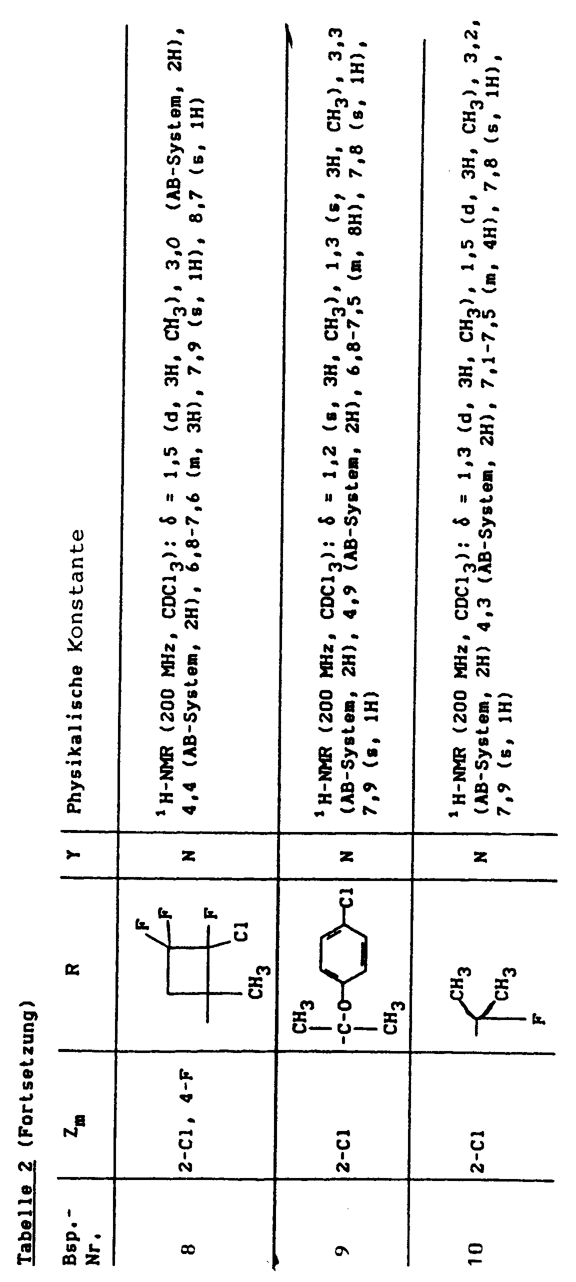

### Herstellung von Ausgangssubstanzen

Nach der im Beispiel angegebenen Methode werden auch die in der folgenden Tabelle 3 aufgeführten Benzylketone der Formel (IV) hergestellt.

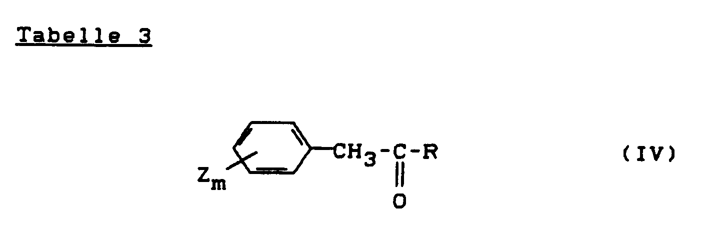

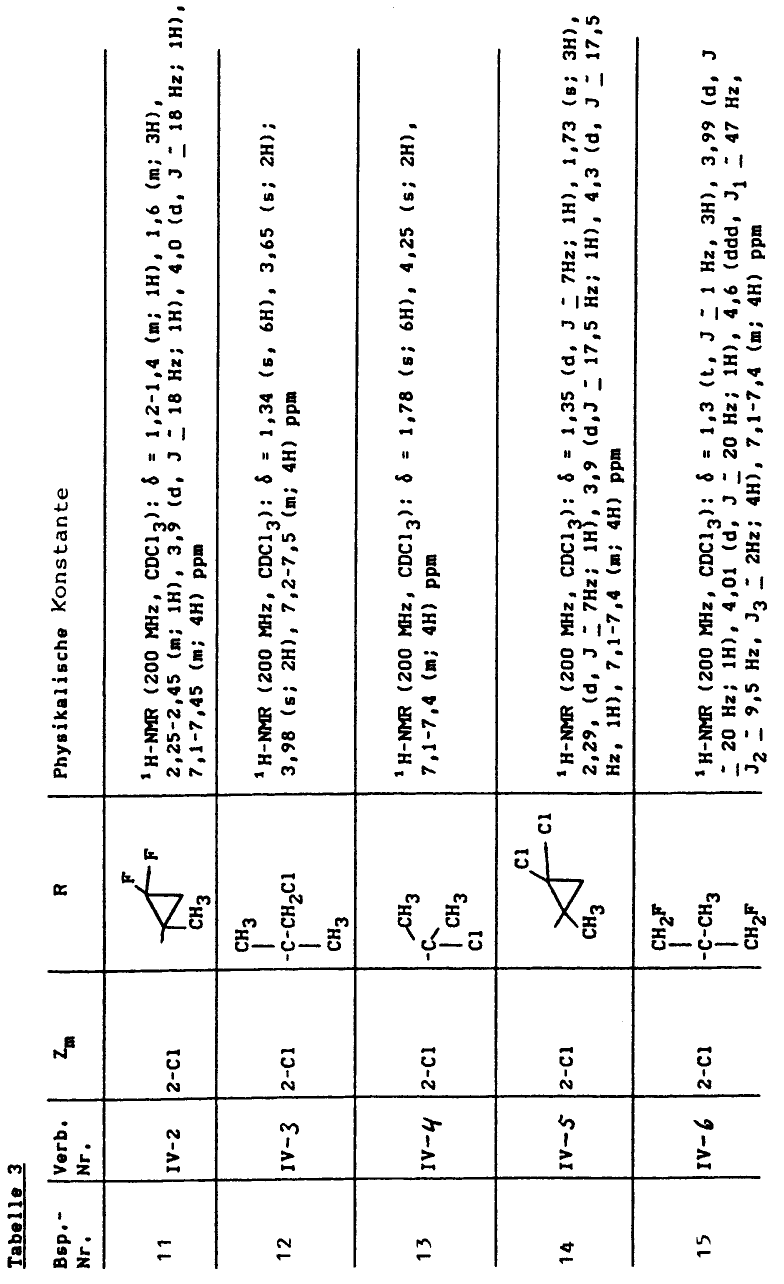

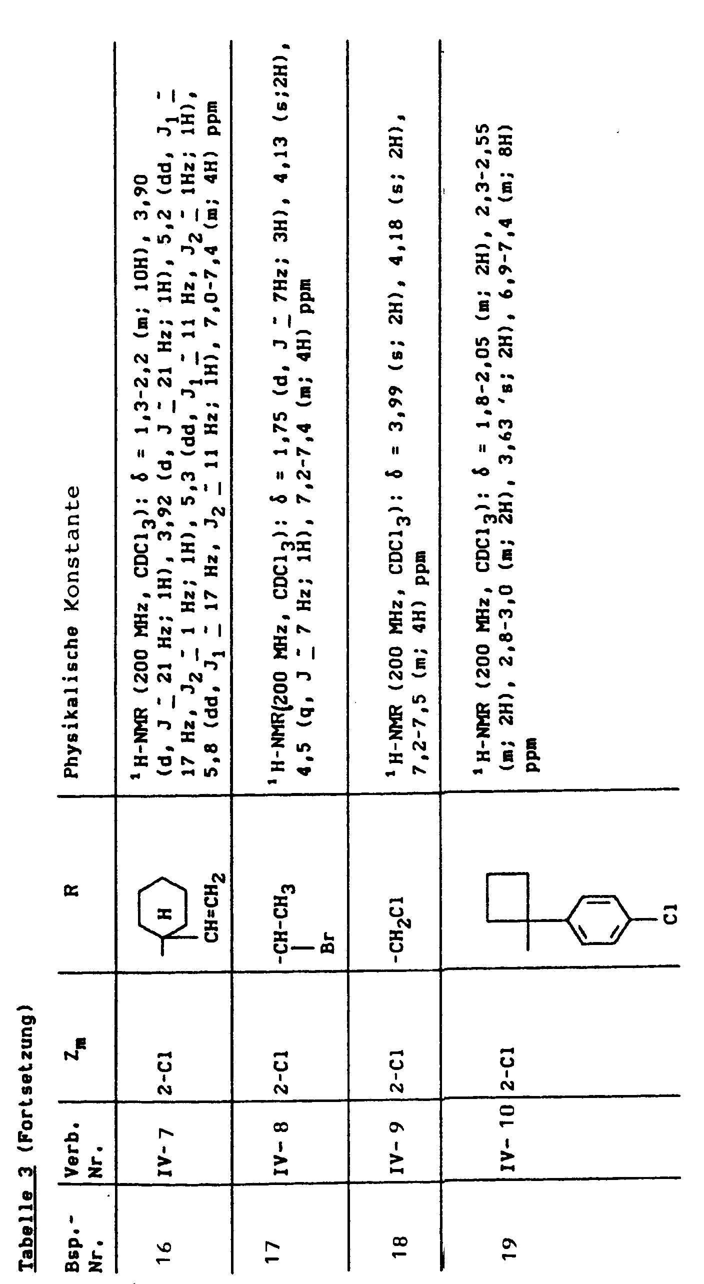

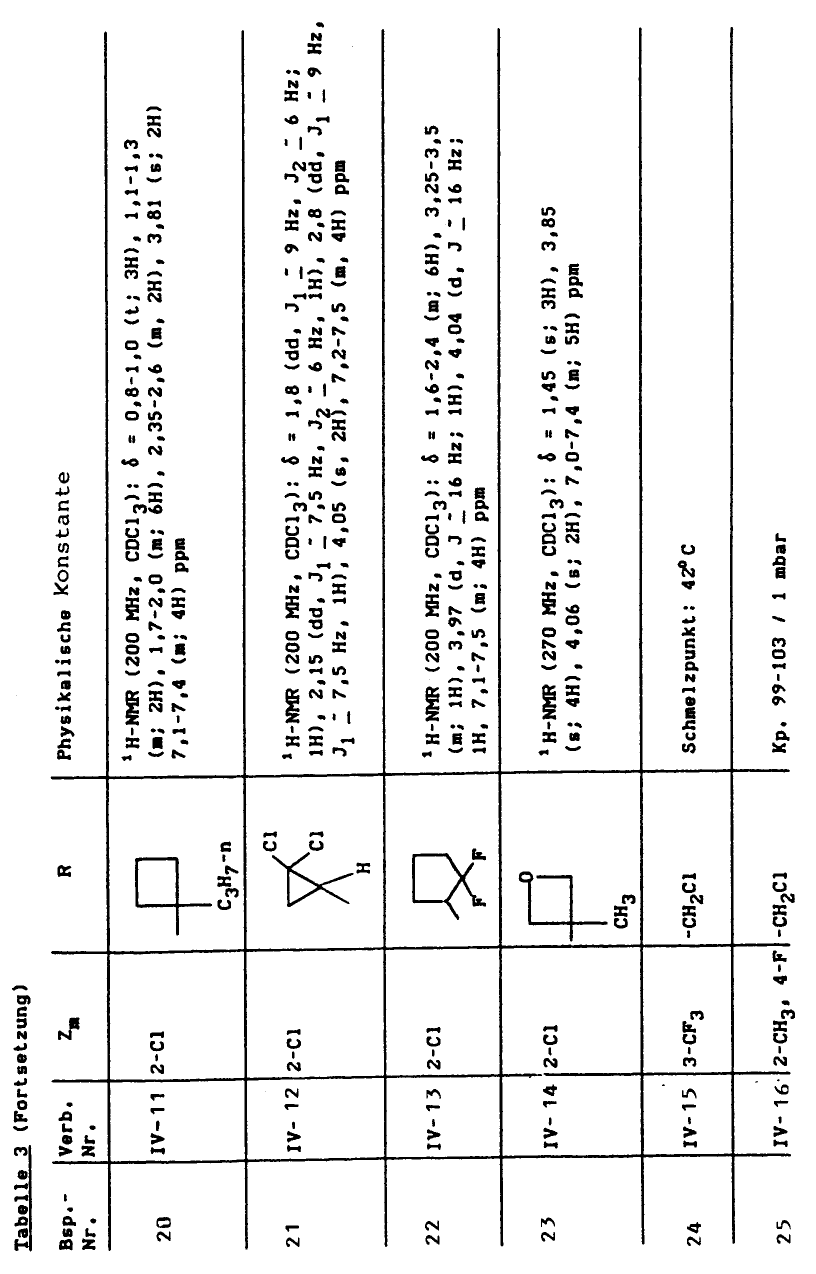

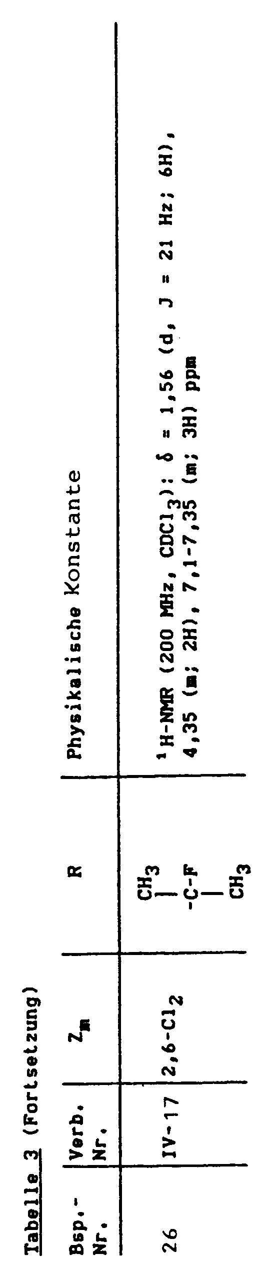

### Beispiel A

### Uncinula-Test (Rebe) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Uncinula necator bestäubt.

Die Pflanzen werden anschließend bei 23 bis 24°C und bei einer relativen Luftfeuchtigkeit von ca. 75 % im Gewächshaus aufgestellt.

14 Tage nach der Inokulation erfolgt die Auswertung.

In diesem Test zeigen die erfindungsgemäßen Stoffe (1), (3), (4) und (9) eine sehr gute Wirksamkeit.

### Beispiel B

### Venturia-Test (Apfel) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100°C relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigen die erfindungsgemäßen Stoffe (3) und (4) eine sehr gute Wirksamkeit.

### Beispiel C

### Botrytis-Test (Buschbohne) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Waser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinera bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

In diesem Test zeigen die erfindungsgemäßen Stoffe (2), (3) und (4) eine sehr gute Wirksamkeit.

### Beispiel D

### Erysiphe-Test (Gerste) / protektiv

- Lösungsmittel:: 100 Gewichtsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

In diesem Test zeigen die erfindungsgemäßen Stoffe (1)-(3), (4), (6), (7) und (9) und (10) eine sehr gute Wirksamkeit.

### Beispiel E

### Pyricularia-Test (Reis) / protektiv

- Lösungsmittel:: 12,5 Gewichtsteile Aceton
- Emulgatgor:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

In diesem Test zeigen die erfindungsgemäßen Stoffe (2), (3), (4) und (6) bis (8) eine sehr gute Wirksamkeit.

### Beispiel F

### Pyricularia-Test (Reis) / systemisch

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

In diesem Test zeigen die erfindungsgemäßen Stoffe (1) bis (3), (4) und (6) bis (8) eine sehr gute Wirksamkeit.

### Beispiel G

### Pellicularia-Test (Reis)

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen in 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

In diesem Test zeigen die erfindungsgemäßen Stoffe (1) bis (3), (4) und (6) bis (8) eine sehr gute Wirksamkeit.

## Patentansprüche

1. Azolyl-propanol-Derivate der Formel 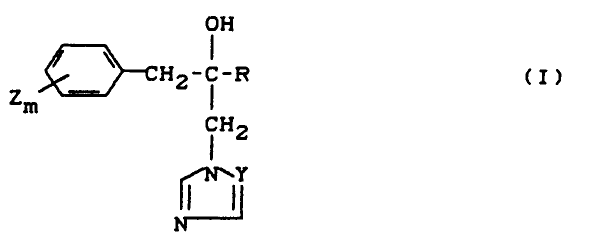 in welcher
R für Halogenalkyl mit 1 bis 18 Kohlenstoffatomen und 1 bis 12 Halogenatomen, Halogenalkenyl mit 2 bis 18 Kohlenstoffatomen und 1 bis 12 Halogenatomen oder einen Rest der Formel 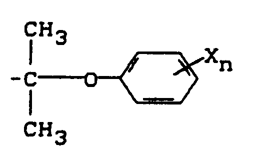 steht, worin
X für Alkyl mit 1 bis 6 Kohlenstoffatomen, Halogen oder Halogenalkyl mit 1 bis 4 Kohlenstoffatomen und 1 bis 5 Halogenatomen steht und
n für die Zahlen 0, 1, 2 oder 3 steht, oder
R für einen Rest der Formel 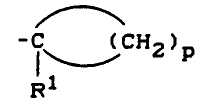 steht, worin
R¹ für n-Propyl, Vinyl oder für gegebenenfalls durch Halogen und/oder Alkyl mit 1 bis 4 Kohlenstoffatomen substituiertes Phenyl steht und
p für die Zahlen 3, 4 oder 5 steht, oder
R für einen Rest der Formel 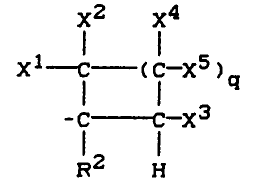 steht, worin
R² für Wasserstoff, Halogen, Methyl oder Ethyl steht,
X¹ für Halogen oder Trifluormethyl steht,
X² für Halogen oder Trifluormethyl steht,
X³ für Wasserstoff oder Halogen steht,
X⁴ für Wasserstoff oder Halogen steht,
X⁵ für Wasserstoff oder Halogen steht und
q für die Zahlen 0, 1 oder 2 steht,
oder
R für einen Rest der Formel 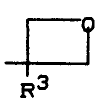 steht, worin
R³ für Alkyl mit 1 bis 4 Kohlenstoffatomen oder für Halogen steht,
Y für Stickstoff oder eine CH-Gruppe steht,
Z für Halogen, Alkyl mit 1 bis 6 Kohlenstoffatomen, Halogenalkyl mit 1 bis 6 Kohlenstoffatomen und 1 bis 13 Halogenatomen, Alkoxy mit 1 bis 6 Kohlenstoffatomen, Halogenalkoxy mit 1 bis 6 Kohlenstoffatomen und 1 bis 13 Halogenatomen, Cyano, gegebenenfalls durch Halogen substituiertes Phenoxy oder für Aryl mit 6 bis 10 Kohlenstoffatomen steht, wobei jeder der Aryl-Reste durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen und/oder Halogenalkoxy mit 1 bis 4 Kohlenstoffatomen und 1 bis 5 Halogenatomen substituiert sein kann, und
m für die Zahlen 0, 1, 2 oder 3 steht,
sowie deren Säureadditions-Salze und Metallsalz-Komplexe.

2. Azolyl-propanol-Derivate der Formel (I) gemäß Anspruch 1, in denen
R für Halogenalkyl mit 1 bis 6 Kohlenstoffatomen und 1 bis 6 Fluor-, Chlor- und/oder Bromatomen oder Halogenalkenyl mit 2 bis 8 Kohlenstoffatomen und 1 bis 6 Fluor-, Chlor- und/oder Bromatomen steht, oder
R für einen Rest der Formel 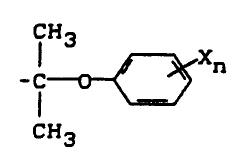 steht, worin
X für Alkyl mit 1 bis 4 Kohlenstoffatomen, Fluor, Chlor oder Halogenalkyl mit 1 oder 2 Kohlenstoffatomen und 1 bis 3 Fluor- und/oder Chloratomen steht und
n für die Zahlen 0, 1 oder 2 steht, oder
R für einen Rest der Formel 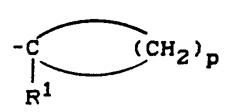 steht, worin
R¹ für n-Propyl, Vinyl oder für gegebenenfalls einfach oder zweifach, gleichartig oder verschieden durch Fluor, Chlor, Methyl und/oder Ethyl substituiertes Phenyl steht und
p für die Zahlen 3, 4 oder 5 steht, oder
R für einen Rest der Formel 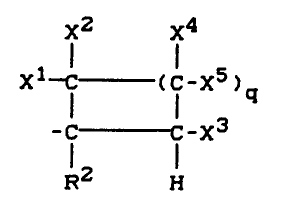 steht, worin
R² für Wasserstoff, Fluor, Chlor, Brom, Methyl oder Ethyl steht,
X¹ für Fluor, Chlor oder Trifluormethyl steht,
X² für Fluor, Chlor oder Trifluormethyl steht,
X³ für Wasserstoff, Fluor oder Chlor steht,
X⁴ für Wasserstoff, Fluor oder Chlor steht,
X⁵ für Wasserstoff, Fluor oder Chlor steht und
q für die Zahlen 0, 1 oder 2 steht, oder
R für einen Rest der Formel 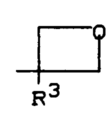 steht, worin
R³ für Methyl, Ethyl, Fluor, Chlor oder Brom steht,
Y für Stickstoff oder eine CH-Gruppe steht,
Z für Fluor, Chlor, Brom, Alkyl mit 1 bis 4 Kohlenstoffatomen, Halogenalkyl mit 1 bis 4 Kohlenstoffatomen und 1 bis 5 Fluor- und/oder Chloratomen, Alkoxy mit 1 bis 4 Kohlenstoffatomen, Halogenalkoxy mit 1 bis 4 Kohlenstoffatomen und 1 bis 5 Fluor- und/oder Chloratomen, Cyano, gegebenenfalls einfach bis dreifach durch Fluor und/oder Chlor substituiertes Phenoxy, für Phenyl oder Naphthyl steht, wobei jeder der beiden zuletzt genannten Reste einfach bis dreifach, gleichartig oder verschieden substituiert sein kann durch Fluor, Chlor, Alkyl mit 1 bis 3 Kohlenstoffatomen, Halogenalkyl mit 1 oder 2 Kohlenstoffatomen und 1 bis 5 Fluor und/oder Chloratomen, Alkoxy mit 1 bis 3 Kohlenstoffatomen oder durch Halogenalkoxy mit 1 oder 2 Kohlenstoffatomen und 1 bis 5 Fluor- und/oder Chloratomen, und
m für die Zahlen 0, 1, 2 oder 3 steht.

3. Verfahren zur Herstellung von Azolyl-propanol-Derivaten der Formel 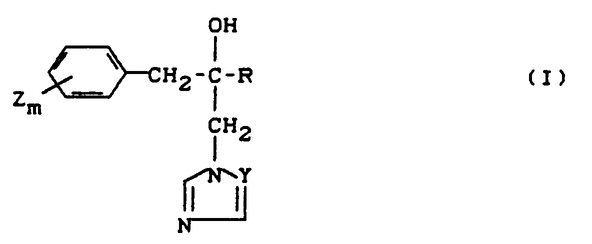 in welcher
R für Halogenalkyl mit 1 bis 18 Kohlenstoffatomen und 1 bis 12 Halogenatomen, Halogenalkenyl mit 2 bis 18 Kohlenstoffatomen und 1 bis 12 Halogenatomen oder einen Rest der Formel 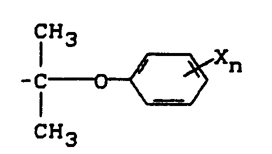 steht, worin
X für Alkyl mit 1 bis 6 Kohlenstoffatomen, Halogen oder Halogenalkyl mit 1 bis 4 Kohlenstoffatomen und 1 bis 5 Halogenatomen steht und
n für die Zahlen 0, 1, 2 oder 3 steht, oder
R für einen Rest der Formel 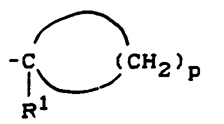 steht, worin
R¹ für n-Propyl, Vinyl oder für gegebenenfalls durch Halogen und/oder Alkyl mit 1 bis 4 Kohlenstoffatomen substituiertes Phenyl steht und
p für die Zahlen 3, 4 oder 5 steht, oder
R für einen Rest der Formel 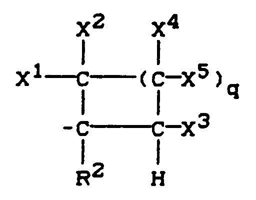 steht, worin
R² für Wasserstoff, Halogen, Methyl oder Ethyl steht,
X¹ für Halogen oder Trifluormethyl steht,
X² für Halogen oder Trifluormethyl steht,
X³ für Wasserstoff oder Halogen steht,
X⁴ für Wasserstoff oder Halogen steht,
X⁵ für Wasserstoff oder Halogen steht und
q für die Zahlen 0, 1 oder 2 steht,
oder
R für einen Rest der Formel 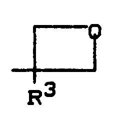 steht, worin
R³ für Alkyl mit 1 bis 4 Kohlenstoffatomen oder für Halogen steht,
Y für Stickstoff oder eine CH-Gruppe steht,
Z für Halogen, Alkyl mit 1 bis 6 Kohlenstoffatomen, Halogenalkyl mit 1 bis 6 Kohlenstoffatomen und 1 bis 13 Halogenatomen, Alkoxy mit 1 bis 6 Kohlenstoffatomen, Halogenalkoxy mit 1 bis 6 Kohlenstoffatomen und 1 bis 13 Halogenatomen, Cyano, gegebenenfalls durch Halogen substituiertes Phenoxy oder für Aryl mit 6 bis 10 Kohlenstoffatomen steht, wobei jeder der Aryl-Reste durch Halogen, Alkyl mit 1 bis 4 Kohlenstoffatomen und 1 bis 5 Halogenatomen, Alkoxy mit 1 bis 4 Kohlenstoffatomen und/oder Halogenalkoxy mit 1 bis 4 Kohlenstoffatomen und 1 bis 5 Halogenatomen substituiert sein kann, und
m für die Zahlen 0, 1, 2 oder 3 steht,
sowie von deren Säureadditions-Salze und Metallsalz-Komplexen, dadurch gekennzeichnet, daß man Oxirane der Formel 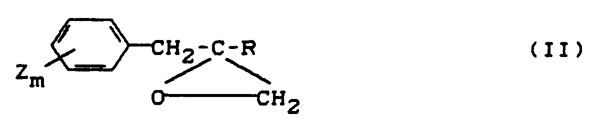 in welcher
R, Z und m die oben angegebenen Bedeutungen haben,
mit Azolen der Formel 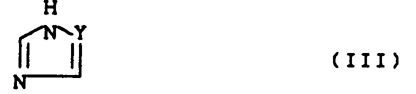 in welcher
Y die oben angegebene Bedeutung hat,
gegebenenfalls in Gegenwart eines Säurebindemittels und in Gegenwart eines Verdünnungsmittels umsetzt
und gegebenenfalls anschließend an die so erhaltenen Verbindungen der Formel (I) eine Säure oder ein Metallsalz addiert.

4. Fungizide Mittel, gekennzeichnet durch einen Gehalt an mindestens einem Azolyl-propanol-Derivat der Formel (I) gemäß Anspruch 1 bzw. an einem Säureadditions-Salz oder Metallsalz-Komplex eines Azolyl-propanol-Derivates der Formel (I).

5. Verwendung von Azolyl-propanol-Derivaten der Formel (I) gemäß Anspruch 1 bzw. von deren Säureadditions-Salzen und Metallsalz-Komplexen zur Bekämpfung von phytopathogenen Pilzen.

6. Verfahren zur Bekämpfung von phytopathogenen Pilzen, dadurch gekennzeichnet, daß man Azolyl-propanol-Derivate der Formel (I) gemäß Anspruch 1 bzw. deren Säureadditions-Salze oder Metallsalz-Komplexe auf die Pilze und/oder deren Lebensraum ausbringt.

7. Verfahren zur Herstellung von fungiziden Mitteln, dadurch gekennzeichnet, daß man Azolyl-propanol-Derivate der Formel (I) gemäß Anspruch 1 bzw. deren Metallsalz-Komplexe oder Säureadditions-Salze mit Streckmitteln und/oder oberflächenaktiven Stoffen vermischt.

8. Oxirane der Formel 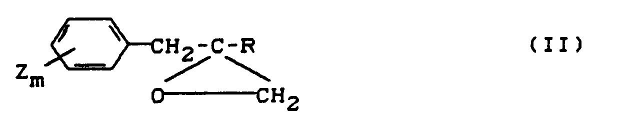 in welcher
R für Halogenalkyl mit 1 bis 18 Kohlenstoffatomen und 1 bis 12 Halogenatomen, Halogenalkenyl mit 2 bis 18 Kohlenstoffatomen und 1 bis 12 Halogenatomen oder einen Rest der Formel 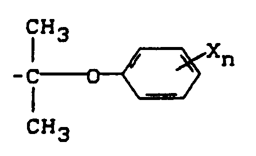 steht, worin
X für Alkyl mit 1 bis 6 Kohlenstoffatomen, Halogen oder Halogenalkyl mit 1 bis 4 Kohlenstoffatomen und 1 bis 5 Halogenatomen steht und
n für die Zahlen 0, 1, 2 oder 3 steht, oder
R für einen Rest der Formel 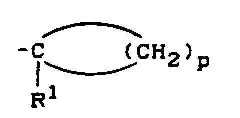 steht, worin
R¹ für n-Propyl, Vinyl oder für gegebenenfalls durch Halogen und/oder Alkyl mit 1 bis 4 Kohlenstoffatomen substituiertes Phenyl steht und
p für die Zahlen 3, 4 oder 5 steht, oder
R für einen Rest der Formel 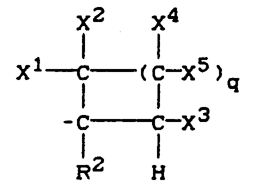 steht, worin
R² für Wasserstoff, Halogen, Methyl oder Ethyl steht,
X¹ für Halogen oder Trifluormethyl steht,
X² für Halogen oder Trifluormethyl steht,
X³ für Wasserstoff oder Halogen steht,
X⁴ für Wasserstoff oder Halogen steht,
X⁵ für Wasserstoff oder Halogen steht und
q für die Zahlen 0, 1 oder 2 steht,
oder
R für einen Rest der Formel 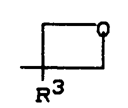 steht, worin
R³ für Alkyl mit 1 bis 4 Kohlenstoffatomen oder für Halogen steht,
Y für Stickstoff oder eine CH-Gruppe steht,
Z für Halogen, Alkyl mit 1 bis 6 Kohlenstoffatomen, Halogenalkyl mit 1 bis 6 Kohlenstoffatomen und 1 bis 13 Halogenatomen, Alkoxy mit 1 bis 6 Kohlenstoffatomen, Halogenalkoxy mit 1 bis 6 Kohlenstoffatomen und 1 bis 13 Halogenatomen, Cyano, gegebenenfalls durch Halogen substituiertes Phenoxy oder für Aryl mit 6 bis 10 Kohlenstoffatomen steht, wobei jeder der Aryl-Reste durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen und/oder Halogenalkoxy mit 1 bis 4 Kohlenstoffatomen und 1 bis 5 Halogenatomen substituiert sein kann, und
m für die Zahlen 0, 1, 2 oder 3 steht.

9. Verfahren zur Herstellung von Oxiranen der Formel 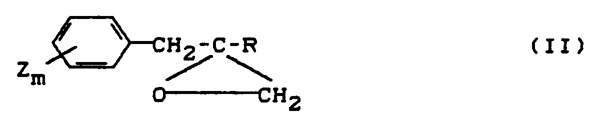 in welcher
R für Halogenalkyl mit 1 bis 18 Kohlenstoffatomen und 1 bis 12 Halogenatomen, Halogenalkenyl mit 2 bis 18 Kohlenstoffatomen und 1 bis 12 Halogenatomen oder einen Rest der Formel 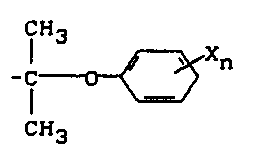 steht, worin
X für Alkyl mit 1 bis 6 Kohlenstoffatomen, Halogen oder Halogenalkyl mit 1 bis 4 Kohlenstoffatomen und 1 bis 5 Halogenatomen steht und
n für die Zahlen 0, 1, 2 oder 3 steht, oder
R für einen Rest der Formel 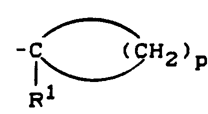 steht, worin
R¹ für n-Propyl, Vinyl oder für gegebenenfalls durch Halogen und/oder Alkyl mit 1 bis 4 Kohlenstoffatomen substituiertes Phenyl steht und
p für die Zahlen 3, 4 oder 5 steht, oder
R für einen Rest der Formel 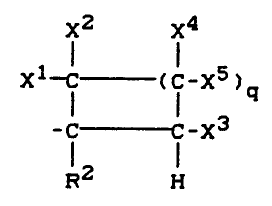 steht, worin
R² für Wasserstoff, Halogen, Methyl oder Ethyl steht,
X¹ für Halogen oder Trifluormethyl steht,
X² für Halogen oder Trifluormethyl steht,
X³ für Wasserstoff oder Halogen steht,
X⁴ für Wasserstoff oder Halogen steht,
X⁵ für Wasserstoff oder Halogen steht, und
q für die Zahlen 0, 1 oder 2 steht, oder
R für einen Rest der Formel 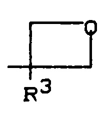 steht, worin
R³ für Alkyl mit 1 bis 4 Kohlenstoffatomen oder für Halogen steht,
Y für Stickstoff oder eine CH-Gruppe steht,
Z für Halogen, Alkyl mit 1 bis 6 Kohlenstoffatomen, Halogenalkyl mit 1 bis 6 Kohlenstoffatomen und 1 bis 13 Halogenatomen, Alkoxy mit 1 bis 6 Kohlenstoffatomen, Halogenalkoxy mit 1 bis 6 Kohlenstoffatomen und 1 bis 13 Halogenatomen, Cyano, gegebenenfalls durch Halogen substituiertes Phenoxy oder für Aryl mit 6 bis 10 Kohlenstoffatomen steht, wobei jeder der Aryl-Reste durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen und/oder Halogenalkoxy mit 1 bis 4 Kohlenstoffatomen und 1 bis 5 Halogenatomen substituiert sein kann, und
m für die Zahlen 0, 1, 2 oder 3 steht,
dadurch gekennzeichnet, daß man Benzyl-ketone der Formel 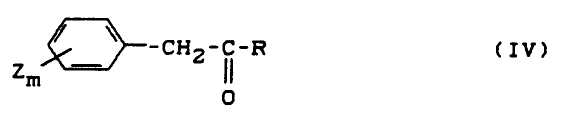 in welcher
R, Z und m die oben angegebenen Bedeutungen haben,
entweder
α) mit Dimethyloxosulfonium-methylid der Formel 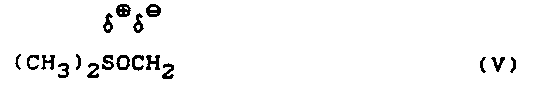 oder
β) mit Dimethylsulfonium-methylid der Formel 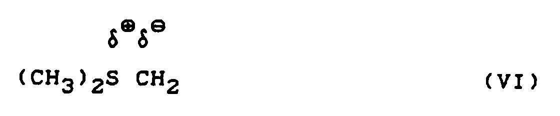 in Gegenwart eines Verdünnungsmittels umsetzt.

## Claims

1. Azolyl-propanol derivatives of the formula 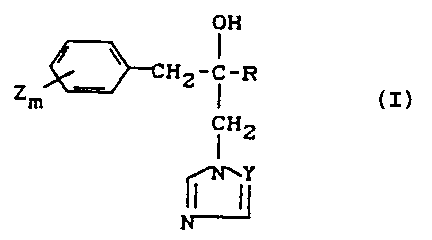 in which
R represents halogenoalkyl having 1 to 18 carbon atoms and 1 to 12 halogen atoms, halogenoalkenyl having 2 to 18 carbon atoms and 1 to 12 halogen atoms, or a radical of the formula 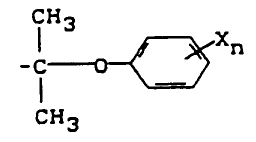 where
X represents alkyl having 1 to 6 carbon atoms, halogen, or halogenoalkyl having 1 to 4 carbon atoms and 1 to 5 halogen atoms, and
n represents the numbers 0, 1, 2 or 3, or
R represents a radical of the formula 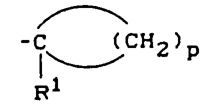 where
R¹ represents n-propyl, vinyl, or phenyl which is optionally substituted by halogen and/or alkyl having 1 to 4 carbon atoms, and
p represents the numbers 3, 4 or 5, or
R represents a radical of the formula 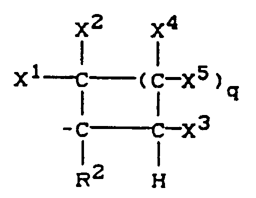 where
R² represents hydrogen, halogen, methyl or ethyl,
X¹ represents halogen or trifluoromethyl,
X² represents halogen or trifluoromethyl,
X³ represents hydrogen or halogen,
X⁴ represents hydrogen or halogen,
X⁵ represents hydrogen or halogen and
q represents the numbers 0, 1 or 2,
or
R represents a radical of the formula 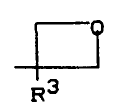 where
R³ represents alkyl having 1 to 4 carbon atoms, or halogen,
Y represents nitrogen or a CH group,
Z represents halogen, alkyl having 1 to 6 carbon atoms, halogenoalkyl having 1 to 6 carbon atoms and 1 to 13 halogen atoms, alkoxy having 1 to 6 carbon atoms, halogenoalkoxy having 1 to 6 carbon atoms and 1 to 13 halogen atoms, cyano, phenoxy which is optionally substituted by halogen, or aryl having 6 to 10 carbon atoms, it being possible for each of the aryl radicals to be substituted by halogen, alkyl having 1 to 4 carbon atoms, halogenoalkyl having 1 to 4 carbon atoms and 1 to 5 halogen atoms, alkoxy having 1 to 4 carbon atoms and/or halogenoalkoxy having 1 to 4 carbon atoms and 1 to 5 halogen atoms, and
m represents the numbers 0, 1, 2 or 3,
and their acid addition salts and metal salt complexes.

2. Azolyl-propanol derivatives of the formula (I) according to Claim 1, in which
R represents halogenoalkyl having 1 to 6 carbon atoms and 1 to 6 fluorine, chlorine and/or bromine atoms, or halogenoalkenyl having 2 to 8 carbon atoms and 1 to 6 fluorine, chlorine and/or bromine atoms, or
R represents a radical of the formula 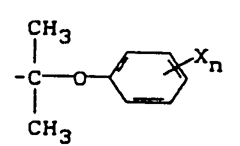 where
X represents alkyl having 1 to 4 carbon atoms, fluorine, chlorine or halogenoalkyl having 1 or 2 carbon atoms and 1 to 3 fluorine and/or chlorine atoms, and
n represents the numbers 0, 1 or 2, or
R represents a radical of the formula 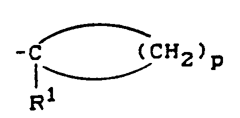 where
R¹ represents n-propyl, vinyl, or phenyl which is optionally monosubstituted or disubstituted by identical or different substituents from the series comprising fluorine, chlorine, methyl and/or ethyl, and
p represents the numbers 3, 4 or 5, or
R represents a radical of the formula 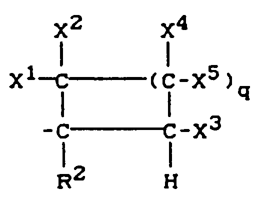 where
R² represents hydrogen, fluorine, chlorine, bromine, methyl or ethyl,
X¹ represents fluorine, chlorine or trifluoromethyl,
X² represents fluorine, chlorine or trifluoromethyl,
X³ represents hydrogen, fluorine or chlorine,
X⁴ represents hydrogen, fluorine or chlorine,
X⁵ represents hydrogen, fluorine or chlorine and
q represents the numbers 0, 1 or 2, or
R represents a radical of the formula 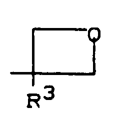 where
R³ represents methyl, ethyl, fluorine, chlorine or bromine,
Y represents nitrogen or a CH group,
Z represents fluorine, chlorine, bromine, alkyl having 1 to 4 carbon atoms, halogenoalkyl having 1 to 4 carbon atoms and 1 to 5 fluorine and/or chlorine atoms, alkoxy having 1 to 4 carbon atoms, halogenoalkoxy having 1 to 4 carbon atoms and 1 to 5 fluorine and/or chlorine atoms, cyano, phenoxy which is optionally monosubstituted to trisubstituted by fluorine and/or chlorine, or represents phenyl or naphthyl, it being possible for each of the two lastmentioned radicals to be monosubstituted to trisubstituted by identical or different substituents from the series comprising fluorine, chlorine, alkyl having 1 to 3 carbon atoms, halogenoalkyl having 1 or 2 carbon atoms and 1 to 5 fluorine and/or chlorine atoms, alkoxy having 1 to 3 carbon atoms or by halogenoalkoxy having 1 or 2 carbon atoms and 1 to 5 fluorine and/or chlorine atoms, and
m represents the numbers 0, 1, 2 or 3.

3. Process for the preparation of azolyl-propanol derivatives of the formula 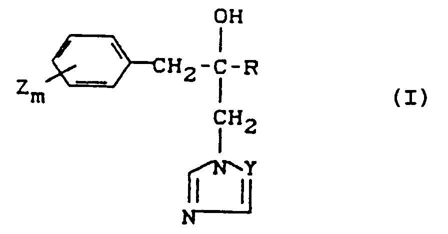 in which
R represents halogenoalkyl having 1 to 18 carbon atoms and 1 to 12 halogen atoms, halogenoalkenyl having 2 to 18 carbon atoms and 1 to 12 halogen atoms, or a radical of the formula 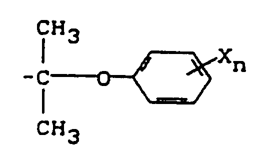 where
X represents alkyl having 1 to 6 carbon atoms, halogen, or halogenoalkyl having 1 to 4 carbon atoms and 1 to 5 halogen atoms, and
n represents the numbers 0, 1, 2 or 3, or
R represents a radical of the formula 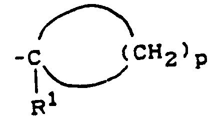 where
R¹ represents n-propyl, vinyl, or phenyl which is optionally substituted by halogen and/or alkyl having 1 to 4 carbon atoms, and
p represents the numbers 3, 4 or 5, or
R represents a radical of the formula 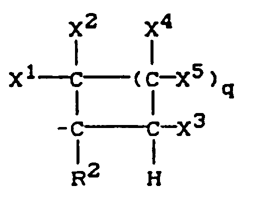 where
R² represents hydrogen, halogen, methyl or ethyl,
X¹ represents halogen or trifluoromethyl,
X² represents halogen or trifluoromethyl,
X³ represents hydrogen or halogen,
X⁴ represents hydrogen or halogen,
X⁵ represents hydrogen or halogen and
q represents the numbers 0, 1 or 2,
or
R represents a radical of the formula 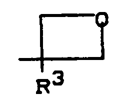 where
R³ represents alkyl having 1 to 4 carbon atoms, or halogen,
Y represents nitrogen or a CH group,
Z represents halogen, alkyl having 1 to 6 carbon atoms, halogenoalkyl having 1 to 6 carbon atoms and 1 to 13 halogen atoms, alkoxy having 1 to 6 carbon atoms, halogenoalkoxy having 1 to 6 carbon atoms and 1 to 13 halogen atoms, cyano, phenoxy which is optionally substituted by halogen, or aryl having 6 to 10 carbon atoms, it being possible for each of the aryl radicals to be substituted by halogen, alkyl having 1 to 4 carbon atoms and 1 to 5 halogen atoms, alkoxy having 1 to 4 carbon atoms and/or halogenoalkoxy having 1 to 4 carbon atoms and 1 to 5 halogen atoms, and
m represents the numbers 0, 1, 2 or 3,
and of their acid addition salts and metal salt complexes, characterised in that oxiranes of the formula 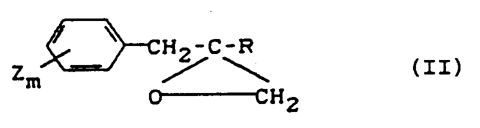 in which
R, Z and m have the abovementioned meanings
are reacted with azoles of the formula 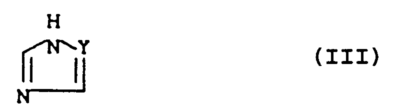 in which
Y has the abovementioned meaning,
if appropriate in the presence of an acid-binding agent and in the presence of a diluent
and, if appropriate, an acid or a metal salt is subsequently added onto the resulting compounds of the formula (I).

4. Fungicidal agents, characterised in that they contain at least one azolyl-propanol derivative of the formula (I) according to Claim 1, or an acid addition salt or metal salt complex of an azolyl-propanol derivative of the formula (I).

5. Use of azolyl-propanol derivatives of the formula (I) according to Claim 1, or of their acid addition salts or metal salt complexes, for combating phytopathogenic fungi.

6. Method of combating phytopathogenic fungi, characterised in that azolyl-propanol derivatives of the formula (I) according to Claim 1 or their acid addition salts or metal salt complexes are applied to the fungi and/or their environment.

7. Process for the preparation of fungicidal agents, characterised in that azolyl-propanol derivatives of the formula (I) according to Claim 1 or their metal salt complexes or acid addition salts are mixed with extenders and/or surface-active substances.

8. Oxiranes of the formula 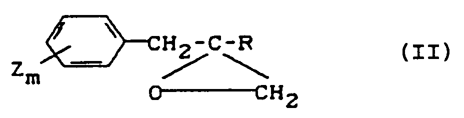 in which
R represents halogenoalkyl having 1 to 18 carbon atoms and 1 to 12 halogen atoms, halogenoalkenyl having 2 to 18 carbon atoms and 1 to 12 halogen atoms, or a radical of the formula 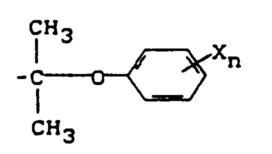 where
X represents alkyl having 1 to 6 carbon atoms, halogen, or halogenoalkyl having 1 to 4 carbon atoms and 1 to 5 halogen atoms, and
n represents the numbers 0, 1, 2 or 3, or
R represents a radical of the formula 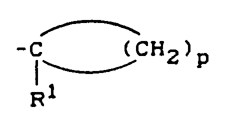 where
R¹ represents n-propyl, vinyl, or phenyl which is optionally substituted by halogen and/or alkyl having 1 to 4 carbon atoms, and
p represents the numbers 3, 4 or 5, or
R represents a radical of the formula 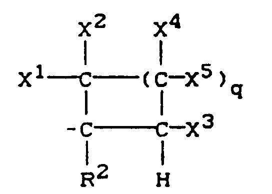 where
R² represents hydrogen, halogen, methyl or ethyl,
X¹ represents halogen or trifluoromethyl,
X² represents halogen or trifluoromethyl,
X³ represents hydrogen or halogen,
X⁴ represents hydrogen or halogen,
X⁵ represents hydrogen or halogen and
q represents the numbers 0, 1 or 2,
or
R represents a radical of the formula 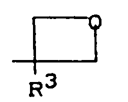 where
R³ represents alkyl having 1 to 4 carbon atoms, or halogen,
Y represents nitrogen or a CH group,
Z represents halogen, alkyl having 1 to 6 carbon atoms, halogenoalkyl having 1 to 6 carbon atoms and 1 to 13 halogen atoms, alkoxy having 1 to 6 carbon atoms, halogenoalkoxy having 1 to 6 carbon atoms and 1 to 13 halogen atoms, cyano, phenoxy which is optionally substituted by halogen, or aryl having 6 to 10 carbon atoms, it being possible for each of the aryl radicals to be substituted by halogen, alkyl having 1 to 4 carbon atoms, halogenoalkyl having 1 to 4 carbon atoms and 1 to 5 halogen atoms, alkoxy having 1 to 4 carbon atoms and/or halogenoalkoxy having 1 to 4 carbon atoms and 1 to 5 halogen atoms, and
m represents the numbers 0, 1, 2 or 3.

9. Process for the preparation of oxiranes of the formula 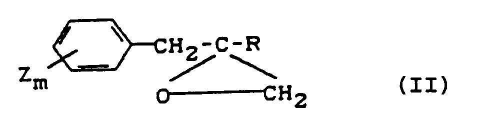 in which
R represents halogenoalkyl having 1 to 18 carbon atoms and 1 to 12 halogen atoms, halogenoalkenyl having 2 to 18 carbon atoms and 1 to 12 halogen atoms, or a radical of the formula 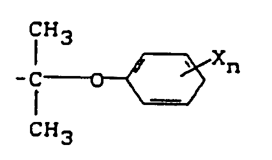 where
X represents alkyl having 1 to 6 carbon atoms, halogen, or halogenoalkyl having 1 to 4 carbon atoms and 1 to 5 halogen atoms, and
n represents the numbers 0, 1, 2 or 3, or
R represents a radical of the formula 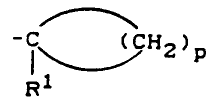 where
R¹ represents n-propyl, vinyl, or phenyl which is optionally substituted by halogen and/or alkyl having 1 to 4 carbon atoms, and
p represents the numbers 3, 4 or 5, or
R represents a radical of the formula 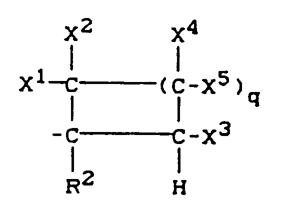 where
R² represents hydrogen, halogen, methyl or ethyl,
X¹ represents halogen or trifluoromethyl,
X² represents halogen or trifluoromethyl,
X³ represents hydrogen or halogen,
X⁴ represents hydrogen or halogen,
X⁵ represents hydrogen or halogen and
q represents the numbers 0, 1 or 2, or
R represents a radical of the formula 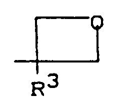 where
R³ represents alkyl having 1 to 4 carbon atoms, or halogen,
Y represents nitrogen or a CH group,
Z represents halogen, alkyl having 1 to 6 carbon atoms, halogenoalkyl having 1 to 6 carbon atoms and 1 to 13 halogen atoms, alkoxy having 1 to 6 carbon atoms, halogenoalkoxy having 1 to 6 carbon atoms and 1 to 13 halogen atoms, cyano, phenoxy which is optionally substituted by halogen, or aryl having 6 to 10 carbon atoms, it being possible for each of the aryl radicals to be substituted by halogen, alkyl having 1 to 4 carbon atoms, halogenoalkyl having 1 to 4 carbon atoms and 1 to 5 halogen atoms, alkoxy having 1 to 4 carbon atoms and/or halogenoalkoxy having 1 to 4 carbon atoms and 1 to 5 halogen atoms, and
m represents the numbers 0, 1, 2 or 3,
characterised in that benzyl ketones of the formula 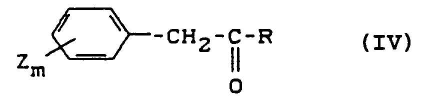 in which
R, Z and m have the abovementioned meanings,
are reacted either
α) with dimethyloxosulphonium methylide of the formula 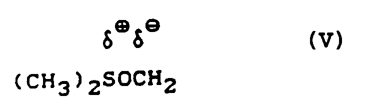 or
β) with dimethylsulphonium methylide of the formula 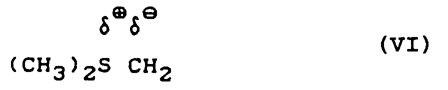 in the presence of a diluent.

## Revendications

1. Dérivés d'azolyl-propanol de formule : 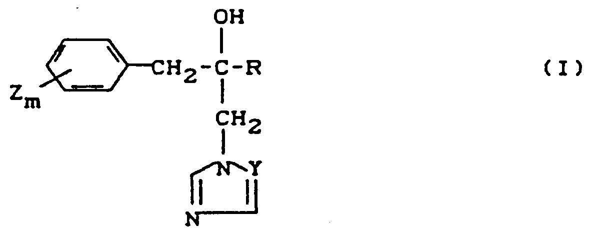 dans laquelle
R est un groupe halogénalkyle ayant 1 à 18 atomes de carbone et 1 à 12 atomes d'halogènes, un groupe halogénalcényle ayant 2 à 18 atomes de carbone et 1 à 12 atomes d'halogènes ou un reste de formule 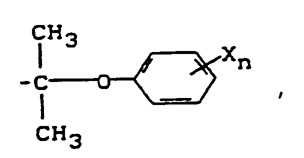 dans laquelle
X est un groupe alkyle ayant 1 à 6 atomes de carbone, un halogène ou un groupe halogénalkyle ayant 1 à 4 atomes de carbone et 1 à 5 atomes d'halogènes, et
n représente les nombres 0, 1, 2 ou 3, ou bien
R représente un reste de formule 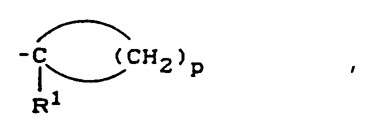 dans laquelle
R¹ est un groupe n-propyle, vinyle ou un groupe phényle éventuellement substitué par un halogène et/ou par un radical alkyle ayant 1 à 4 atomes de carbone,
et
p représente les noires 3, 4 ou 5, ou bien
R est un reste de formule 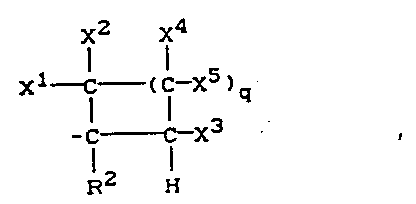 dans laquelle
R² est de l'hydrogène, un halogène, un groupe méthyle ou éthyle,
X¹ est un halogène ou un groupe trifluorométhyle,
X² est un halogène ou un groupe trifluorométhyle,
X³ est de l'hydrogène ou un halogène,
X⁴ est de l'hydrogène ou un halogène,
X⁵ est de l'hydrogène ou un halogène, et
q représente les noires 0, 1 ou 2,
ou bien
R est un reste de formule 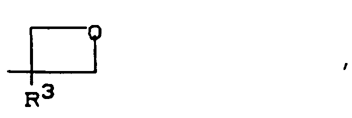 dans laquelle
R³ est un groupe alkyle ayant 1 à 4 atomes de carbone ou un halogène,
Y est de l'azote ou un groupe CH,
Z est un halogène, un groupe alkyle ayant 1 à 6 atomes de carbone, un groupe halogénalkyle ayant 1 à 6 atomes de carbone et 1 à 13 atomes d'halogènes, un groupe alkoxy ayant 1 à 6 atomes de carbone, un groupe halogénoxy ayant 1 à 6 atomes de carbone et 1 à 13 atomes d'halogènes, un groupe cyano, un groupe phénoxy éventuellement substitué par un halogène ou un reste aryle ayant 6 à 10 atomes de carbone, chacun des restes aryle pouvant être substitué par un halogène, un radical alkyle ayant 1 à 4 atomes de carbone, halogénalkyle ayant 1 à 4 atomes de carbone et 1 à 5 atomes d'halogènes, alkoxy ayant 1 à 4 atomes de carbone et/ou halogénalkoxy ayant 1 à 4 atomes de carbone et 1 à 5 atomes d'halogènes, et
m représente les nombres 0, 1, 2 ou 3,
ainsi que leurs sels d'addition d'acides et leurs complexes de sels métalliques.

2. Dérivés d'azolyl-propanol de formule (I) suivant la revendication 1, dans lesquels
R représente un groupe halogénalkyle ayant 1 à 6 atomes de carbone et 1 à 6 atomes de fluor, de chlore et/ou de brome ou un groupe halogénalkyle ayant 2 à 8 atomes de carbone et 1 à 6 atomes de fluor, de chlore et/ou de brome, ou bien
R représente un reste de formule 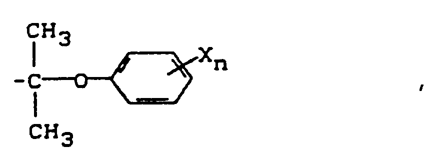 dans laquelle
X est un groupe alkyle ayant 1 à 4 atomes de carbone, du fluor, du chlore ou un groupe halogénalkyle ayant 1 ou 2 atomes de carbone et 1 à 3 atomes de fluor et/ou de chlore et
n représente les nombres 0, 1 ou 2, ou bien
R représente un reste de formule 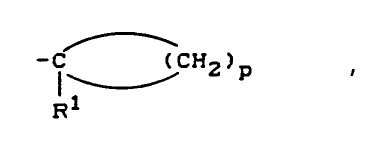 dans laquelle
R¹ est un groupe n-propyle, vinyle ou un groupe phényle éventuellement substitué une ou deux fois identiques ou différentes par du fluor, du chlore, un radical méthyle et/ou éthyle, et
p représente les nombres 3, 4 ou 5, ou bien
R représente un reste de formule 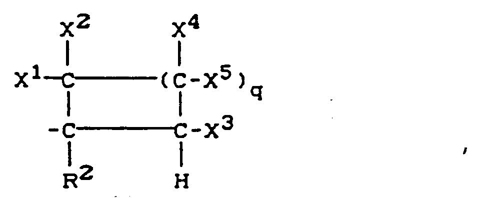 dans laquelle
R² est de l'hydrogène, du fluor, du chlore, du brome, un groupe méthyle ou éthyle,
X¹ est du fluor, du chlore ou un groupe trifluorométhyle,
X² est du fluor, du chlore ou un groupe trifluorométhyle,
X³ est de l'hydrogène, du fluor ou du chlore,
X⁴ est de l'hydrogène, du fluor ou du chlore,
X⁵ est de l'hydrogène, du fluor ou du chlore et
q représente les nombres 0, 1 ou 2, ou bien
R est un reste de formule 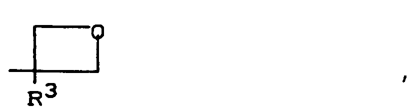 dans laquelle
R³ est un groupe méthyle, éthyle, du fluor, du chlore ou du brome,
Y est de l'azote ou un groupe CH,
Z représente du fluor, du chlore, du brome, un groupe alkyle ayant 1 à 4 atomes de carbone, un groupe halogénalkyle ayant 1 à 4 atomes de carbone et 1 à 5 atomes de fluor et/ou de chlore, un groupe alkoxy ayant 1 a 4 atomes de carbone, un groupe halogénalkoxy ayant 1 à 4 atomes de carbone et 1 à 5 atomes de fluor et/ou de chlore, un groupe cyano, un groupe phénoxy substitué le cas échéant 1 à 3 fois par du fluor et/ou du chlore, un reste phényle ou naphtyle, chacun des deux restes mentionnés en dernier lieu pouvant être substitué 1 à 3 fois identiques ou différentes par du fluor, du chlore, un radical alkyle ayant 1 à 3 atomes de carbone, halogénalkyle ayant 1 ou 2 atomes de carbone et 1 à 5 atomes de fluor et/ou de chlore, alkoxy ayant 1 à 3 atomes de carbone ou par un radical halogénalkoxy ayant 1 ou 2 atomes de carbone et 1 à 5 atomes de fluor et/ou de chlore, et
m représente les nombres 0, 1, 2 ou 3.

3. Procédé de production de dérivés d'azolyl-propanol de formule 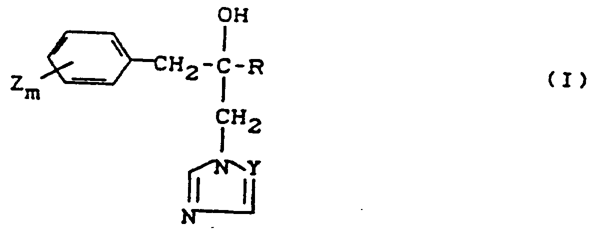 dans laquelle
R est un groupe halogénalkyle ayant 1 à 18 atomes de carbone et 1 à 12 atomes d'halogènes, un groupe halogénalcényle ayant 2 à 18 atomes de carbone et 1 à 12 atomes d'halogènes ou un reste de formule 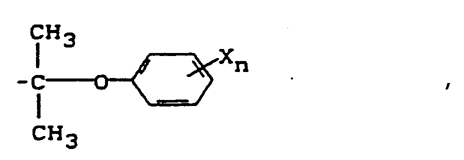 dans laquelle
X est un groupe alkyle ayant 1 à 6 atomes de carbone, un halogène ou un groupe halogénalkyle ayant 1 à 4 atomes de carbone et 1 à 5 atomes d'halogènes, et
n représente les nombres 0, 1, 2 ou 3,
ou bien
R représente un reste de formule 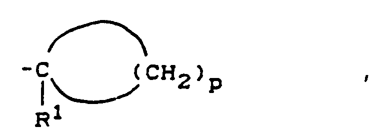 dans laquelle
R¹ est un groupe n-propyle, vinyle ou un groupe phényle éventuellement substitué par un halogène et/ou par un radical alkyle ayant 1 à 4 atomes de carbone,
et
p représente les nombres 3, 4 ou 5, ou bien
R est un reste de formule 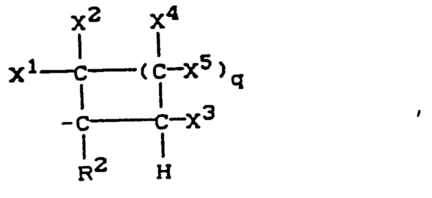 dans laquelle
R² est de l'hydrogène, un halogène, un groupe méthyle ou éthyle,
X¹ est un halogène ou un groupe trifluorométhyle,
X² est un halogène ou un groupe trifluorométhyle,
X³ est de l'hydrogène ou un halogène,
X⁴ est de l'hydrogène ou un halogène,
X⁵ est de l'hydrogène ou un halogène, et
q représente les nombres 0, 1 ou 2,
ou bien
R est un reste de formule 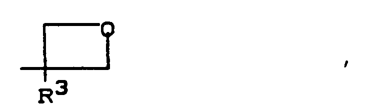 dans laquelle
R³ est un groupe alkyle ayant 1 à 4 atomes de carbone ou un halogène,
Y est de l'azote ou un groupe CH,
Z est un halogène, un groupe alkyle ayant 1 à 6 atomes de carbone, un groupe halogénalkyle ayant 1 à 6 atomes de carbone et 1 à 13 atomes d'halogènes, un groupe alkoxy ayant 1 à 6 atomes de carbone, un groupe halogénoxy ayant 1 à 6 atomes de carbone et 1 à 13 atomes d'halogènes, un groupe cyano, un groupe phénoxy éventuellement substitué par un halogène ou un reste aryle ayant 6 à 10 atomes de carbone, chacun des restes aryle pouvant être substitué par un halogène, un radical alkyle ayant 1 à 4 atomes de carbone et 1 à 5 atomes d'halogènes, alkoxy ayant 1 à 4 atomes de carbone et/ou halogénalkoxy ayant 1 à 4 atomes de carbone et 1 à 5 atomes d'halogènes, et
m représente les nombres 0, 1, 2 ou 3,
ainsi que de leurs sels d'addition d'acides et de leurs complexes de sels métalliques, caractérisé en ce qu'on fait réagir des oxirannes de formule 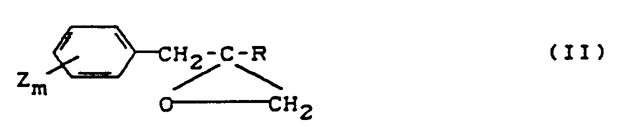 dans laquelle
R, Z et m ont les définitions indiquées ci-dessus,
avec des azoles de formule 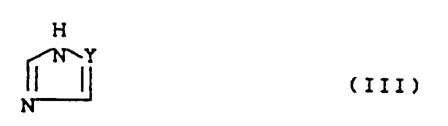 dans laquelle
Y a la définition indiquée ci-dessus,
le cas échéant en présence d'un accepteur d'acide et en la présence d'un diluant,
puis le cas échéant, on additionne un acide ou un sel métallique sur les composés de formule (I) ainsi obtenus.

4. Compositions fongicides, caractérisées par une teneur en au moins un dérivé d'azolyl-propanol de formule (I) suivant la revendication 1 ou d'un sel d'addition d'acide ou d'un complexe de sel métallique d'un dérivé d'azolyl-propanol de formule (I).

5. Utilisation de dérivés d'azolyl-propanol de formule (I) suivant la revendication 1 ou de leurs sels d'addition d'acides et de leurs complexes de sels métalliques pour combattre des champignons phytopathogènes.

6. Procédé pour combattre des champignons phytopathogènes, caractérisé en ce qu'on applique des dérivés d'azolyl-propanol de formule (I) suivant la revendication 1 ou leurs sels d'addition d'acides ou leurs complexes de sels métalliques sur les champignons et/ou sur leur milieu.

7. Procédé de préparation de compositions fongicides, caractérisé en ce qu'on mélange des dérivés d'azolyl-propanol de formule (I) suivant la revendication 1 ou leurs complexes de sels métalliques ou leurs sels d'addition d'acides avec des diluants et/ou des agents tensioactifs.

8. Oxirannes de formule 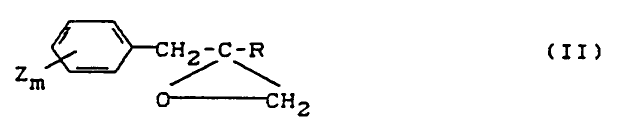 dans laquelle
R est un groupe halogénalkyle ayant 1 à 18 atomes de carbone et 1 à 12 atomes d'halogènes, un groupe halogénalcényle ayant 2 à 18 atomes de carbone et 1 à 12 atomes d'halogènes ou un reste de formule 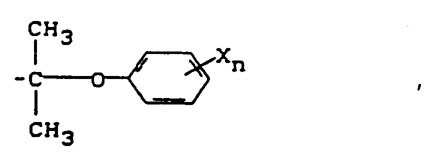 dans laquelle
X est un groupe alkyle ayant 1 à 6 atomes de carbone, un halogène ou un groupe halogénalkyle ayant 1 à 4 atomes de carbone et 1 à 5 atomes d'halogènes et
n représente les nombres 0, 1, 2 ou 3,
ou bien
R représente un reste de formule 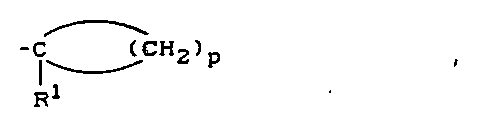 dans laquelle
R¹ est un groupe n-propyle, vinyle ou un groupe phényle éventuellement substitué par un halogène et/ou par un groupe alkyle ayant 1 à 4 atomes de carbone et
p représente les nombres 3, 4 ou 5, ou bien
R est un reste de formule 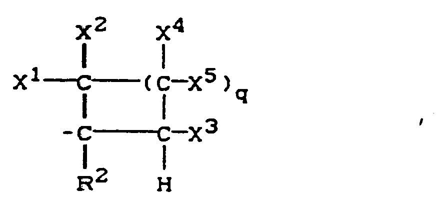 dans laquelle
R² est de l'hydrogène, un halogène, un groupe méthyle ou éthyle,
X¹ est un halogène ou un groupe trifluorométhyle,
X² est un halogène ou un groupe trifluorométhyle,
X³ est de l'hydrogène ou un halogène,
X⁴ est de l'hydrogène ou un halogène,
X⁵ est de l'hydrogène ou un halogène, et
q représente les nombres 0, 1 ou 2,
ou bien
R représente un reste de formule 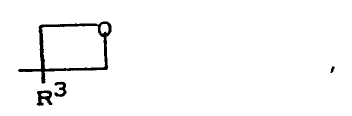 dans laquelle
R³ est un groupe alkyle ayant 1 à 4 atomes de carbone ou un halogène,
Y représente de l'azote ou un groupe CH,
Z est un halogène, un groupe alkyle ayant 1 à 6 atomes de carbone, un groupe halogénalkyle ayant 1 à 6 atomes de carbone et 1 à 13 atomes d'halogènes, un groupe alkoxy ayant 1 à 6 atomes de carbone, un groupe halogénalkoxy ayant 1 à 6 atomes de carbone et 1 à 13 atomes d'halogènes, un groupe cyano, un groupe phénoxy éventuellement substitué par un halogène ou un reste aryle ayant 6 à 10 atomes de carbone, chacun des restes aryle pouvant être substitué par un halogène, un radical alkyle ayant 1 à 4 atomes de carbone, un radical halogénalkyle ayant 1 à 4 atomes de carbone et 1 à 5 atomes d'halogènes, un radical alkoxy ayant 1 à 4 atomes de carbone et/ou un radical halogénalkoxy ayant 1 à 4 atomes de carbone et 1 à 5 atomes d'halogènes, et
m représente les nombres 0, 1, 2 ou 3.

9. Procédé de production d'oxirannes de formule 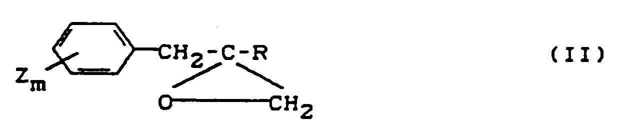 dans laquelle
R est un groupe halogénalkyle ayant 1 à 18 atomes de carbone et 1 à 12 atomes d'halogènes, un groupe halogénalcényle ayant 2 à 18 atomes de carbone et 1 à 12 atomes d'halogènes ou un reste de formule 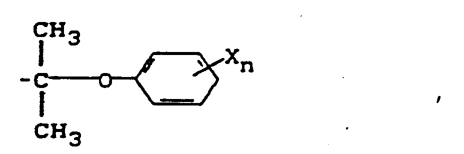 dans laquelle
X est un groupe alkyle ayant 1 à 6 atomes de carbone, un halogène ou un groupe halogénalkyle ayant 1 à 4 atomes de carbone et 1 à 5 atomes d'halogènes et
n représente les nombres 0, 1, 2 ou 3,
ou bien
R représente un reste de formule 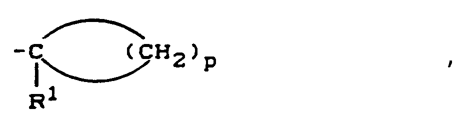 dans laquelle
R¹ est un groupe n-propyle, vinyle ou un groupe phényle éventuellement substitué par un halogène et/ou par un groupe alkyle ayant 1 à 4 atomes de carbone et
p représente les nombres 3, 4 ou 5, ou bien
R est un reste de formule 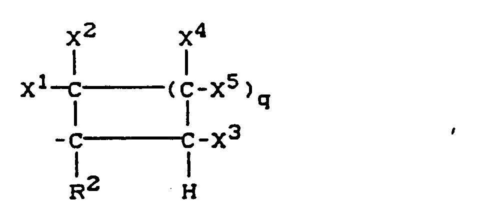 dans laquelle
R² est de l'hydrogène, un halogène, un groupe méthyle ou éthyle,
X¹ est un halogène ou un groupe trifluorométhyle,
X² est un halogène ou un groupe trifluorométhyle,
X³ est de l'hydrogène ou un halogène,
X⁴ est de l'hydrogène ou un halogène,
X⁵ est de l'hydrogène ou un halogène, et
q représente les nombres 0, 1 ou 2,
ou bien
R représente un reste de formule 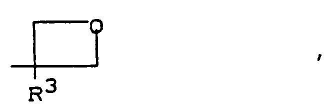 dans laquelle
R³ est un groupe alkyle ayant 1 à 4 atomes de carbone ou un halogène,
Y représente de l'azote ou un groupe CH,
Z est un halogène, un groupe alkyle ayant 1 à 6 atomes de carbone, un groupe halogénalkyle ayant 1 à 6 atomes de carbone et 1 à 13 atomes d'halogènes, un groupe alkoxy ayant 1 à 6 atomes de carbone, un groupe halogénalkoxy ayant 1 à 6 atomes de carbone et 1 à 13 atomes d'halogènes, un groupe cyano, un groupe phénoxy éventuellement substitué par un halogène ou un reste aryle ayant 6 à 10 atomes de carbone, chacun des restes aryle pouvant être substitué par un halogène, un radical alkyle ayant 1 à 4 atomes de carbone, un radical halogénalkyle ayant 1 à 4 atomes de carbone et 1 à 5 atomes d'halogènes, un radical alkoxy ayant 1 à 4 atomes de carbone et/ou un radical halogénalkoxy ayant 1 à 4 atomes de carbone et 1 à 5 atomes d'halogènes, et
m représente les nombres 0, 1, 2 ou 3,
caractérisé en ce qu'on fait réagir des benzyl-cétones de formule 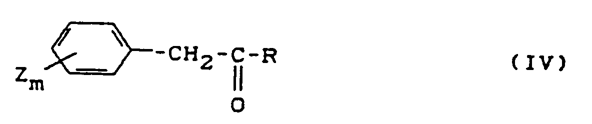 dans laquelle
R, Z et m ont les définitions indiquées ci-dessus,
soit
α) avec le méthylure de diméthyloxosulfonium de formule 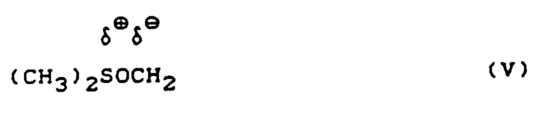 soit
β) avec le méthylure de diméthylsulfonium de formule 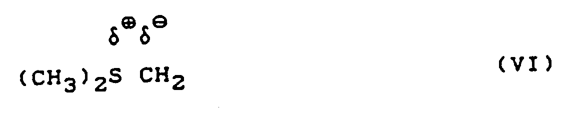 en présence d'un diluant.
